(19) **Europäisches Patentamt / European Patent Office / Office européen des brevets**

(11) **EP 4 567 115 A1**

(12) **EUROPEAN PATENT APPLICATION**
published in accordance with Art. 153(4) EPC

(43) Date of publication:
**11.06.2025 Bulletin 2025/24**

(21) Application number: **23850060.7**

(22) Date of filing: **31.07.2023**

(51) International Patent Classification (IPC):
*C12N 15/31* (2006.01)     *C12P 21/00* (2006.01)
*C12P 21/02* (2006.01)     *C07K 14/245* (2006.01)

(52) Cooperative Patent Classification (CPC):
**C07K 14/195; C07K 14/245; C12P 21/00;**
**C12P 21/02**

(86) International application number:
**PCT/JP2023/028022**

(87) International publication number:
**WO 2024/029506 (08.02.2024 Gazette 2024/06)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **03.08.2022 JP 2022124329**

(71) Applicant: **OSAKA UNIVERSITY**
**Suita-shi**
**Osaka 565-0871 (JP)**

(72) Inventors:
• **AOKI, Wataru**
 **Suita-shi, Osaka 565-0871 (JP)**
• **KOSAKA, Yuishin**
 **Kyoto-shi, Kyoto 606-8501 (JP)**

(74) Representative: **Müller-Boré & Partner**
**Patentanwälte PartG mbB**
**Friedenheimer Brücke 21**
**80639 München (DE)**

Remarks:
The complete document including Reference Table(s) and the Sequence Listing(s) can be downloaded from the EPO website

(54) **METHOD FOR PRODUCING RIBOSOME IN CELL-FREE PROTEIN SYNTHESIS SYSTEM**

(57) One purpose of the present disclosure is to provide a method for producing a ribosomal large subunit in a cell-free protein synthesis system by using a one-pot reaction. A method for producing a ribosomal large subunit in a cell-free protein synthesis system, wherein the method for producing a ribosomal large subunit includes a step for assembling ribosomal large subunit proteins and ribosomal large subunit RNAs in a cell-free protein synthesis system to form a ribosomal large subunit, at least one of the ribosomal large subunit proteins in the cell-free protein synthesis system being generated from a template nucleic acid that encodes a ribosomal large subunit protein.

FIG. 1

## Description

Technical Field

The present disclosure relates to a method for producing a ribosomal large subunit and/or a ribosomal small subunit in a cell-free protein synthesis system by using a one-pot reaction.

Background Art

The ribosome is a molecular complex that reads mRNA genetic information to synthesize a protein. The ribosome is formed using a small subunit (SSU) and a large subunit (LSU). For example, the 70S ribosome of *E. coli* is formed using 30S subunit (SSU) composed of 21 proteins and one RNA (16S rRNA), and 50S subunit (LSU) composed of 33 proteins and two RNAs (23S rRNA and 5S rRNA).

If the catalytic activity of the ribosome can be modified by introducing a mutation into a constituent element of the ribosome, it is expected to lead to realization of, for instance, improvement in translation efficiency of a protein, efficient synthesis of a non-natural protein containing a non-natural amino acid, and efficient synthesis of a non-natural polymer containing an ester bond, a thioester bond, a glycoside bond, and the like. However, since the ribosome is an essential factor of life and introduction of a mutation into the ribosome is directly linked to cell death in most cases, it is extremely difficult to produce a cell containing a ribosome with a mutation introduced. Therefore, conventionally, a technique for reconstituting a ribosome *in vitro* has been developed.

For example, NPL 1 reports that a ribosome can be reconstituted by using transcribed rRNAs and r-proteins while transcribing ribosomal RNAs (rRNAs) *in vitro* in the presence of all ribosomal proteins (r-proteins) separated from the 70S ribosome. In such a method, it is easy to introduce a mutation into an rRNA, but there is a disadvantage that a mutation cannot be introduced into an r-protein.

In addition, NPL 2 reports that an SSU can be reconstituted by simultaneously performing transcription of 16S rRNA and transcription and translation of one SSU r-protein in the presence of 20 different SSU r-proteins *in vitro.* Such a technique has the disadvantage that it takes a lot of time and effort because the 20 different SSU r-proteins have to be expressed, purified and then prepared. Meanwhile, a mutation can be introduced into an rRNA and one of SSU r-proteins to be transcribed and translated. However, to introduce a mutation to any of the 20 different SSU r-proteins directly added to the reaction system, r-protein mutants need to be prepared individually. This causes a disadvantage that a ribosome cannot be readily produced that has a mutation introduced into each of two or more different r-proteins. It is extremely difficult to generate an r-protein(s) from a template nucleic acid(s) to produce a ribosome because transcription/translation/assembly needs to be highly coordinated. In NPL 2, generation of two or more different SSU r-proteins from template nucleic acids has not been realized. Further, the ribosomal LSU is known to be more complex in structure and difficult to reconstitute than the ribosomal SSU. Hence, it is not considered that the technique of NPL 2 regarding the reconstitution of the ribosomal SSU can be diverted to the reconstitution of the ribosomal LSU.

Citation List

Non Patent Literature

NPL 1: Michael C Jewett et al., In vitro integration of ribosomal RNA synthesis, ribosome assembly, and translation, Mol Syst Biol. 2013 Jun 25;9:678. doi: 10.1038/msb.2013.31.

NPL 2: Shimojo, M. et al. In vitro reconstitution of functional small ribosomal subunit assembly for comprehensive analysis of ribosomal elements in E. coli. Commun Biol 3, 142 (2020).

Summary of Invention

Technical Problem

One purpose of the present disclosure is to provide a method for producing a ribosomal LSU, a ribosomal SSU, or a ribosome in a cell-free protein synthesis system by using a one-pot reaction.

Solution to Problem

The present inventors conducted intensive research to establish a technology for producing a ribosomal LSU by using a one-pot reaction in a cell-free protein synthesis system. Here, the present inventors have found that in a ribosomal LSU production method comprising the step of assembling LSU r-proteins and LSU rRNAs in a cell-free protein synthesis system to form a ribosomal LSU, the ribosomal LSU can be easily produced in a one-pot reaction by generating at least one of the LSU r-proteins from a template nucleic acid(s) in the cell-free protein synthesis system.

In addition, the present inventors conducted intensive research to establish a technology for producing a ribosomal SSU by using a one-pot reaction in a cell-free protein synthesis system. Here, the present inventors have found that in a ribosomal SSU production method comprising the step of assembling SSU r-proteins and an SSU rRNA in a cell-free protein synthesis system to form a ribosomal SSU, the ribosomal SSU can be easily produced in a one-pot reaction by generating at least two of the SSU r-proteins from template nucleic acids in the cell-free protein synthesis system.

Further, the present inventors conducted intensive research to establish a technology for producing a ribosome by using a one-pot reaction in a cell-free protein synthesis system. Here, the present inventors have found that in a ribosome production method comprising the step of assembling LSU r-proteins and LSU rRNAs and assembling SSU r-proteins and an SSU rRNA in a cell-free protein synthesis system to form a ribosome, the ribosome can be easily produced in a one-pot reaction by generating at least one of the LSU r-proteins or the SSU r-proteins from a template nucleic acid(s) in the cell-free protein synthesis system.

Furthermore, a mutation can be easily introduced into the r-protein template nucleic acid by conventional means. Therefore, in the method using the r-protein template nucleic acid as a starting material, it is possible to easily produce a ribosomal SSU and/or LSU with a mutation introduced by adding the mutation to the template nucleic acid. The present disclosure has been completed by conducting further studies based on the findings.

An embodiment of the present disclosure provides a method for producing a ribosomal LSU and a method for producing a polymer by using the ribosomal LSU as described below.

Item 1-1. A method for producing a ribosomal LSU in a cell-free protein synthesis system, the method comprising the step of:

> assembling LSU r-proteins and LSU rRNAs in a cell-free protein synthesis system to form a ribosomal LSU, wherein in the cell-free protein synthesis system, at least one of the LSU r-proteins is generated from a template nucleic acid encoding each corresponding LSU r-protein.

Item 1-2. The production method according to item 1-1, wherein at least one of the LSU rRNAs is generated from a template DNA encoding each corresponding LSU rRNA in the cell-free protein synthesis system.

Item 1-3. The production method according to item 1-1 or 1-2, wherein the ribosomal LSU is derived from *E. coli.*

Item 1-4. The production method according to item 1-3, wherein the ribosomal large subunit proteins constituting the ribosomal LSU are free of 50S r-protein L11 and consists of 32 or less 50S r-proteins.

Item 1-5. The production method according to any one of item 1-1 to 1-4, wherein any of the LSU rRNAs is mutated to impart drug resistance.

Item 1-6. The production method according to item 1-5, wherein the ribosomal LSU is derived from *E. coli,* and 23S rRNA is mutated to A2058U.

Item 1-7. The production method according to any one of items 1-1 to 1-6, wherein all the LSU r-proteins to be assembled are generated in the cell-free protein synthesis system from template nucleic acids encoding the LSU r-proteins.

Item 1-8. The production method according to any one of items 1-1 to 1-7, wherein the template nucleic acids encoding the LSU r-proteins each have a concentration of 1 fM to 1 $\mu$M in the cell-free protein synthesis system.

Item 1-9. The production method according to item 1-2, wherein the template DNAs encoding the LSU rRNAs each have a concentration of 1 fM to 1 $\mu$M in the cell-free protein synthesis system.

Item 1-10. The production method according to any one of items 1-1 to 1-9, wherein the cell-free protein synthesis system comprises 1 pM to 1 M of a ribosome for translating an mRNA transcribed from a template DNA encoding an LSU r-protein.

Item 1-11. The production method according to any one of items 1-1 to 1-10, wherein the cell-free protein synthesis system comprises polysucrose.

Item 1-12. The production method according to item 1-11, wherein the polysucrose is contained in an amount of 0.1 to 20 wt/v% in the cell-free protein synthesis system.

Item 1-13. A method for producing a polymer in a cell-free synthesis system, the method comprising:

> a first step of assembling LSU r-proteins and LSU rRNAs in a cell-free protein synthesis system to produce a ribosomal LSU; and
> a second step of synthesizing a polymer from a template nucleic acid encoding a sequence of the polymer in the cell-free synthesis system containing a ribosome having the ribosomal LSU obtained in the first step, wherein in the cell-free protein synthesis system of the first step, at least one of the LSU r-proteins is generated from a template nucleic acid encoding each corresponding LSU r-protein in the cell-free protein synthesis system.

In addition, another embodiment of the present disclosure provides a method for producing a ribosomal SSU and a method for producing a polymer by using the ribosomal SSU as described below.

Item 2-1. A method for producing a ribosomal SSU in a cell-free protein synthesis system, the method comprising the step of:

assembling SSU r-proteins and an SSU rRNA in a cell-free protein synthesis system to form a ribosomal SSU, wherein in the cell-free protein synthesis system, at least two of the SSU r-proteins are generated from template nucleic acids encoding the corresponding SSU r-proteins.

Item 2-2. The production method according to item 2-1, wherein the SSU rRNA is generated from a template DNA encoding the SSU rRNA in the cell-free protein synthesis system.

Item 2-3. The production method according to item 2-1 or 2-2, wherein the ribosomal SSU is derived from *E. coli.*

Item 2-4. The production method according to any one of items 2-1 to 2-3, wherein an anti-Shine-Dalgarno sequence of the SSU r-RNA is set so as to be able to recognize a Shine-Dalgarno sequence that is not recognized by an anti-Shine-Dalgarno sequence of an SSU r-RNA of a ribosome used for translation in the cell-free protein synthesis system.

Item 2-5. The production method according to any one of item 2-1 to 2-4, wherein the SSU rRNA is mutated to impart drug resistance.

Item 2-6. The production method according to item 2-5, wherein the ribosomal SSU is derived from *E. coli,* and 16S rRNA is mutated to C1192U.

Item 2-7. The production method according to any one of item 2-1 to 2-6, wherein at least one of the SSU r-proteins is mutated to impart drug resistance.

Item 2-8. The production method according to item 2-7, wherein the ribosomal SSU is derived from *E. coli,* and 30S r-protein S12 is mutated to K43T.

Item 2-9. The production method according to any one of items 2-1 to 2-8, wherein all the SSU r-proteins to be assembled are generated in the cell-free protein synthesis system from template nucleic acids encoding the SSU r-proteins.

Item 2-10. The production method according to any one of items 2-1 to 2-9, wherein the template nucleic acids encoding the SSU r-proteins each have a concentration of 1 fM to 1 $\mu$M in the cell-free protein synthesis system.

Item 2-11. The production method according to item 2-2, wherein the template DNA encoding the SSU rRNA has a concentration of 1 fM to 1 $\mu$M in the cell-free protein synthesis system.

Item 2-12. The production method according to any one of items 2-1 to 2-11, wherein the cell-free protein synthesis system comprises 1 pM to 1 M of a ribosome for translating an mRNA transcribed from a template DNA encoding an SSU r-protein.

Item 2-13. The production method according to any one of items 2-1 to 2-12, wherein the cell-free protein synthesis system comprises polysucrose.

Item 2-14. The production method according to item 2-13, wherein the polysucrose is contained in an amount of 0.1 to 20 wt/v% in the cell-free protein synthesis system.

Item 2-15. A method for producing a polymer in a cell-free synthesis system, the method comprising:

a first step of assembling SSU r-proteins and an SSU rRNA in a cell-free protein synthesis system to produce a ribosomal SSU; and
a second step of synthesizing a polymer from a template nucleic acid encoding a sequence of the polymer in the cell-free synthesis system containing a ribosome having the ribosomal SSU obtained in the first step, wherein in the cell-free protein synthesis system of the first step, at least two of the SSU r-proteins are generated from template nucleic acids encoding the corresponding SSU r-proteins.

Further, still another embodiment of the present disclosure provides a method for producing a ribosome and a method for producing a polymer by using the ribosome as described below.

Item 3-1. A method for producing a ribosome in a cell-free protein synthesis system, the method comprising the step of:

assembling LSU r-proteins and LSU rRNAs and assembling SSU r-proteins and an SSU rRNA in a cell-free protein synthesis system to form a ribosome, wherein in the cell-free protein synthesis system, at least one of the LSU r-proteins or the SSU r-proteins is generated from a template nucleic acid encoding each corresponding protein.

Item 3-2. The production method according to item 3-1, wherein the ribosome is derived from *E. coli.*

Item 3-3. The production method according to item 3-2, wherein the LSU r-proteins constituting the ribosomal LSU are free of 50S r-protein L11 and consists of 32 or less 50S r-proteins.

Item 3-4. The production method according to any one of item 3-1 to 3-3, wherein any of the LSU rRNAs is mutated to impart drug resistance.

Item 3-5. The production method according to item 3-4, wherein the ribosomal LSU is derived from *E. coli,* and 23S ribosomal RNA is mutated to A2058U.

Item 3-6. The production method according to any one of items 3-1 to 3-5, wherein an anti-Shine-Dalgarno sequence of the SSU r-RNA is set so as to be able to recognize a Shine-Dalgarno sequence that is not recognized by an anti-Shine-Dalgarno sequence of an SSU r-RNA of a ribosome used for translation in the cell-free protein synthesis system.

Item 3-7. The production method according to any one of item 3-1 to 3-6, wherein the SSU rRNA is mutated to impart drug resistance.

Item 3-8. The production method according to item 3-7, wherein the ribosomal SSU is derived from *E. coli,* and 16S rRNA is mutated to C1192U.

Item 3-9. The production method according to any one of item 3-1 to 3-8, wherein at least one of the SSU r-proteins is mutated to impart drug resistance.

Item 3-10. The production method according to item 3-9, wherein the ribosomal SSU is derived from *E. coli,* and 30S r-protein S12 is mutated to K43T.

Item 3-11. The production method according to any one of items 3-1 to 3-10, wherein all the LSU r-proteins to be assembled are generated in the cell-free protein synthesis system from template nucleic acids encoding the LSU r-proteins.

Item 3-12. The production method according to any one of items 3-1 to 3-11, wherein all the SSU r-proteins to be assembled are generated in the cell-free protein synthesis system from template nucleic acids encoding the SSU r-proteins.

Item 3-13. The production method according to any one of items 3-1 to 3-12, wherein at least one of the LSU rRNAs or the SSU rRNA is generated in the cell-free protein synthesis system from a template DNA encoding each corresponding rRNA.

Item 3-14. The production method according to any one of items 3-1 to 3-13, wherein the template nucleic acids encoding the r-proteins each have a concentration of 1 fM to 1 $\mu$M in the cell-free protein synthesis system.

Item 3-15. The production method according to item 3-13, wherein the template DNAs encoding the rRNAs each have a concentration of 1 fM to 1 $\mu$M in the cell-free protein synthesis system.

Item 3-16. The production method according to any one of items 3-1 to 3-15, wherein the cell-free protein synthesis system comprises 1 pM to 1 M of a ribosome for translating an mRNA transcribed from a template DNA encoding an r-protein.

Item 3-17. The production method according to any one of items 3-1 to 3-16, wherein the cell-free protein synthesis system comprises polysucrose.

Item 3-18. The production method according to item 3-17, wherein the polysucrose is contained in an amount of 0.1 to 20 wt/v% in the cell-free protein synthesis system.

Item 3-19. A method for producing a polymer in a cell-free synthesis system, the method comprising:

a first step of assembling LSU r-proteins and LSU rRNAs and assembling SSU r-proteins and an SSU rRNA in a cell-free protein synthesis system to form a ribosome; and
a second step of synthesizing a polymer from a template nucleic acid encoding a sequence of the polymer in the cell-free synthesis system containing the ribosome obtained in the first step, wherein
in the cell-free protein synthesis system of the first step, at least one of the LSU r-proteins or the SSU r-proteins is generated from a template nucleic acid encoding each corresponding protein.

Advantageous Effects of Invention

An embodiment of the production method of the present disclosure enables the formation of a ribosomal LSU containing at least one LSU r-protein generated using each template nucleic acid in a one-pot reaction in a cell-free protein synthesis system. In addition, another embodiment of the production method of the present disclosure enables the formation of a ribosomal SSU containing at least two SSU r-proteins generated using template nucleic acids in a one-pot reaction in a cell-free protein synthesis system. Further, still another embodiment of the production method of the present disclosure enables the formation of a ribosome containing at least one r-protein generated using each template nucleic acid in a one-pot reaction in a cell-free protein synthesis system.

According to the production method of the present disclosure, one or two or more r-proteins are generated from each template nucleic acid to form a ribosomal LSU, a ribosomal SSU, or a ribosome. Thus, it is easy to introduce a mutation into the r-protein by mutating the template nucleic acid. In addition, according to the production method of the present disclosure, it is also possible to use a template DNA encoding rRNA as a source of rRNAs for a cell-free protein synthesis system used to form a ribosomal LSU, a ribosomal SSU, or a ribosome. This can make it easy to introduce a mutation into the rRNA by mutating the template DNA. As such, the production method of the present disclosure makes it possible to freely mutate an r-protein and/or an rRNA. This makes it possible to develop a ribosomal LSU, ribosomal SSU or ribosome with various catalytic activities. Further, provided is a ribosomal LSU, a ribosomal SSU, or a ribosome having catalytic activity modified using the production method of the present disclosure. This also accelerates technological development such as improvement in translation efficiency of peptides and proteins, improvement in translation efficiency of peptides and proteins having low translation efficiency in naturally occurring ribosomes, synthesis of peptides and proteins containing non-natural amino acids, synthesis of peptides and proteins containing non-natural monomers other than non-natural amino acids, and synthesis of non-natural polymers having bonds, other than peptide bonds, such as ester bonds and thioester bonds.

In recent years, attention has been paid to a non-natural polymer containing a non-natural monomer such as a non-natural amino acid, a non-natural polymer having a bond other than a peptide bond, and the like as pharmaceutical compounds. Here, a ribosomal LSU, a ribosomal SSU, or a ribosome having catalytic activity modified using the production method of the present disclosure may be provided to promote development of these pharmaceutical compounds.

Brief Description of Drawings

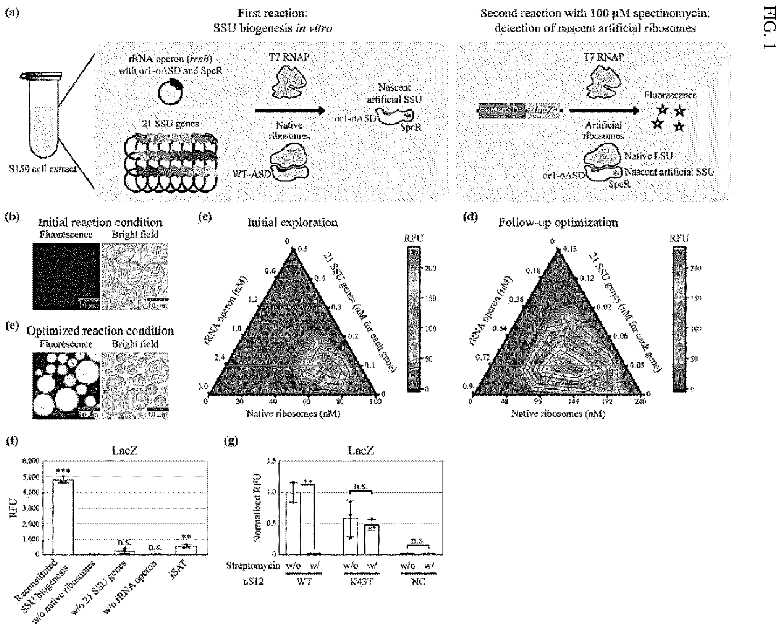

[Fig. 1] Fig. 1a is an experimental scheme for ribosomal SSU formation *in vitro.* Fig. 1b shows the results of performing a femtoliter droplet assay by setting the conditions of the first reaction to 20 nM of native ribosomes (endogenous ribosomes), 0.25 nM each of the 21 different plasmids each containing one of the 21 different SSU r-protein genes, and 1 nM of a plasmid containing an rRNA operon encoding 16S rRNA with orl-oASD and C1192U spectinomycin resistance (SpcR) to form an SSU. In Fig. 1b, the scale bar is 10 $\mu$m. Fig. 1c shows the results of a simplex-lattice design search for the concentrations of native ribosomes, plasmids containing the SSU r-protein genes, and a plasmid containing the rRNA operon with orl-oASD and SpcR in the first reaction. In Fig. 1c, RFU is the average of the relative fluorescence of the fluoresce-emitting droplets. Fig. 1d shows the results of further searching for each of the optimal concentrations of the native ribosomes, the plasmids containing the SSU r-protein genes, and the plasmid containing the rRNA operon with orl-oASD and SpcR in the first reaction. In Fig 1d, RFU is the average of the relative fluorescence of the fluoresce-emitting droplets. Fig. 1e is a representative photomicrograph of droplets in the case of forming SSUs under the optimized conditions and performing a femtoliter droplet assay. In Fig. 1e, the scale bar is 10 $\mu$m. Fig. 1f shows the results of forming SSUs under the optimized conditions (80 nM native ribosome, 0.05 nM each of the 21 different plasmids each containing one of the 21 different SSU r-protein genes, and 0.3 nM of the plasmid containing the rRNA operon with orl-oASD and SpcR) and detecting the translational activity of SSUs by a bulk assay. In Fig. 1f, "Reconstructed SSU biogenesis" represents optimized conditions, "w/o native ribosomes" represents conditions obtained by removing native ribosomes from the optimized conditions, "w/o 21 SSU genes" represents conditions obtained by removing the 21 different plasmids each containing one of 21 different SSU r-protein genes from the optimized conditions, "w/o rRNA operon" represents conditions obtained by removing the plasmid containing the rRNA operon with orl-oASD and SpcR from the optimized conditions, and "iSAT" represents a case where an SSU is reconstituted by iSAT. In Fig. 1f, RFU is a relative fluorescence unit, data is a mean $\pm$ SD (n = 3), *** is P < 0.001, ** is P < 0.01, and n.s. is without a significant difference (Dunnett's test vs. "w/o native ribosomes"). Fig. 1g shows the results of detecting the translational activity of SSU by a bulk assay while using, instead of the 30S r-protein S12-encoding gene, a plasmid containing a gene encoding a K43T mutant of 30S r-protein S12, which mutant confers streptomycin resistance to ribosomes in the first reaction, to form the SSU by mixing only spectinomycin or spectinomycin and streptomycin as antibiotics in the second reaction. In Fig. 1g, RFU is a relative fluorescence unit, NC is a negative control without using the 21 plasmids containing respective 21 SSU r-protein genes, data is a mean $\pm$ SD (n = 3), ** is P < 0.01, n.s. is without a significant difference (Welch's t-test). Here, w/o represents "without", and w/ represents "with".

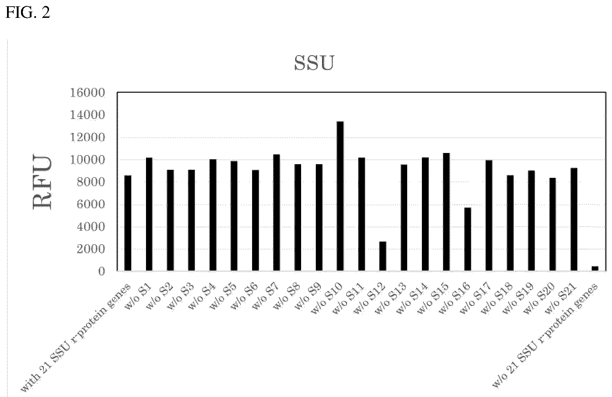

[Fig. 2] Fig. 2 shows the results of ribosomal SSU formation *in vitro* using 21 or 20 different plasmids each containing one of 21 or 20 different SSU r-protein genes and a plasmid containing an rRNA operon with orl-oASD and SpcR. In Fig. 2, "with 21 SSU r-protein genes" represents a condition under which 21 different plasmids each containing one of 21 different SSU r-protein genes were added, "w/o S1" represents a condition under which 20 different plasmids each containing one of 20 different SSU r-protein genes excluding S1 gene were added (the same applies to other S2 to S 21), and "w/o 21 SSU r-protein genes" represents a condition under which the 21 different plasmids each containing one of 21 SSU r-protein genes were not added. In Fig. 2, RFU is a relative fluorescent unit.

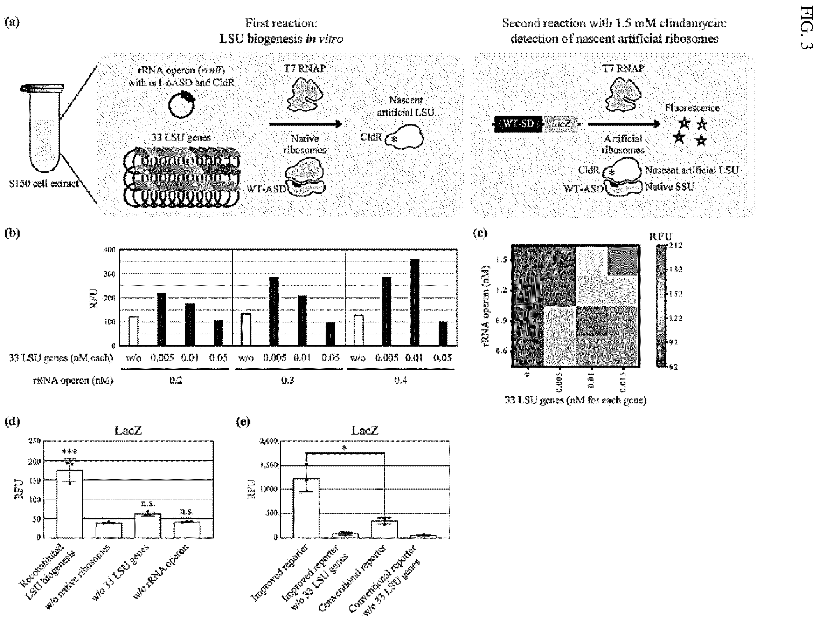

[Fig. 3] Fig. 3a is an experimental scheme for ribosomal LSU formation *in vitro.* Fig. 3b shows the results of searching for each optimal concentration in the formation of LSU while fixing the concentration of native ribosomes to 80 nM, the concentration of each of 33 different plasmids each containing one of the 33 different LSU r-protein genes to the range of 0-0.05 nM, and the concentration of a plasmid containing an rRNA operon encoding 16S rRNA with orl-oASD and SpcR and 23S rRNA with A2058U clindamycin resistance (CldR) to the range of 0.2-0.4 nM in the first reaction. In Fig 3b, RFU is the relative fluorescence unit, and w/o indicates "without". Fig. 3c shows the results of further searching for each of the optimal concentrations of the plasmids containing the LSU r-protein genes and the plasmid containing the rRNA operon with orl-oASD and SpcR and CldR in the first reaction. Fig. 3d shows the results of measuring a reporter signal by a bulk assay in the case of the conditions under which the first reaction was optimized (80 nM native ribosome, 0.01 nM each of the 33 different plasmids each containing one of the 33 different LSU r-protein genes, and 0.9 nM of the plasmid containing an rRNA operon with orl-oASD and SpcR and CldR). In Fig. 3d, "Reconstituted LSU biogenesis" represents optimized conditions, "w/o native ribosomes" represents conditions obtained by removing native ribosomes from the optimized conditions, "w/o 33 LSU genes" represents conditions obtained by removing the 33 different plasmids each containing one of 33 different LSU r-protein genes from the optimized conditions, and "w/o rRNA operon" represents conditions obtained by removing the rRNA operon with orl-oASD, SpcR, and CLdR from the optimized conditions. In Fig. 3d, RFU is a relative fluorescence unit, data is a mean ± SD (n = 3), *** is $P < 0.001$, and n.s. is without a significant difference (Dunnett's test vs. "w/o native ribosomes"). Fig. 3e shows the results of measuring an LSU signal by using the pT7PCONS_EpsA20_wtSD_lacZ reporter or the pT7_wtSD_LacZ reporter. The "improved reporter" is data obtained by using the pT7PCONS_EpsA20_wtSD_lacZ reporter, and the "conventional reporter" is data obtained by using the pT7_wtSD_LacZ reporter. In Fig. 3e, RFU is a relative fluorescence unit, data is a mean ± SD (n = 3), and * is $P < 0.05$ (Welch's t-test).

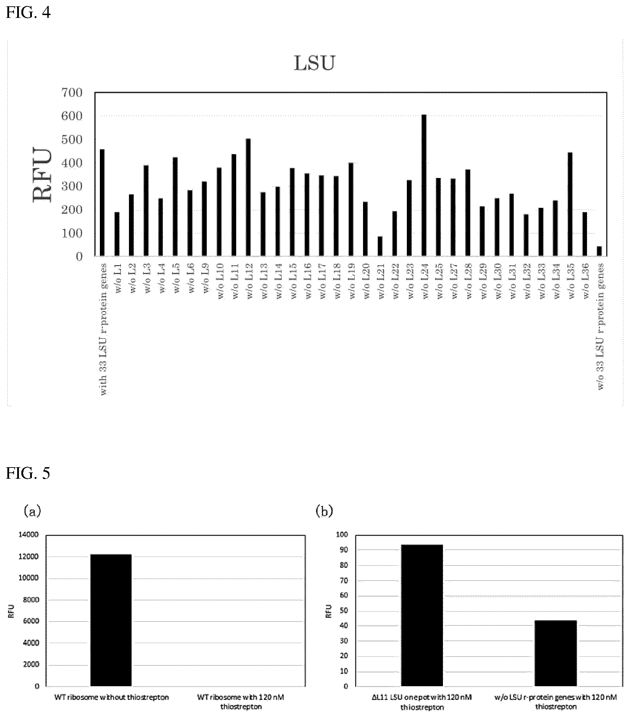

[Fig. 4] Fig. 4 shows the results of ribosomal LSU formation *in vitro* using each of 33 or 32 different plasmids each containing one of 33 or 32 different LSU r-protein genes and the plasmid containing an rRNA operon with orl-oASD, SpcR, and CldR. In Fig. 4, "with 33 LSU r-protein genes" represents a condition under which 33 different plasmids each containing one of 33 different LSU r-protein genes were added, "w/o L1" represents a condition under which 32 different plasmids each containing one of 32 different LSU r-protein genes excluding L1 gene were added (the same applies to other LSU r-protein genes), and "w/o 33 LSU r-protein genes" represents a condition under which the 33 different plasmids each containing one of 33 different LSU r-protein genes were not added. In addition, in Fig. 4, RFU is a relative fluorescent unit.

[Fig. 5] Fig. 5a shows the results of checking the translational activity of native ribosomes by adding native ribosomes in the first reaction and performing a bulk assay in the presence or absence of thiostrepton in the second reaction during the formation of ribosomal LSU *in vitro.* In Fig. 5a, RFU is a relative fluorescent unit, "WT ribosome without thiostrepton" is a condition under which thiostrepton was not added in the second reaction, and "WT ribosome with 120 nM thiostrepton" is a condition under which thiostrepton was added in the second reaction. Fig. 5b shows the results of measuring a ribosomal LSU signal by performing the first reaction using or not using 32 different plasmids each containing one of 32 different LSU r-protein genes excluding 50S r-protein L11 to form a ribosomal LSU, and performing a bulk assay in the presence of thiostrepton in the second reaction. In Fig. 5b, RFU is a relative fluorescence unit, "ΔL11 LSU one pot with 120 nM thiostrepton" is a condition using 32 different plasmids each containing one of 32 different LSU r-protein genes excluding 50S r-protein L11, and "w/o LSU r-protein genes with 120 nM thiostrepton" is a condition without using 33 different plasmids each containing one of 33 different LSU r-protein genes.

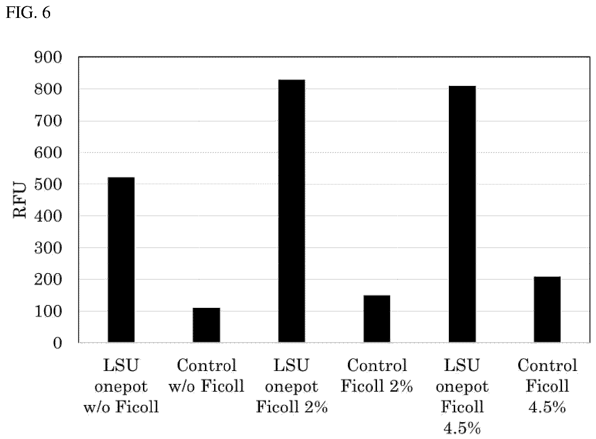

[Fig. 6] Fig. 6 shows the results of adding Ficoll 400 at each concentration of 0, 2, or 4.5 w/v% in the first reaction and forming a ribosomal LSU *in vitro.* In Fig. 6, the "LSU onepot w/o Ficoll" is a condition without adding Ficoll 400; "Control w/o Ficoll" is a condition without using Ficoll 400 or 33 different plasmids each containing one of 33 different LSU r-protein genes (negative control); "LSU onepot Ficoll 2%" is a condition with 2% Ficoll 400 added; "Control Ficoll 2%" is a condition with 2% Ficoll 400 added and without using 33 different plasmids each containing one of 33 different LSU r-protein genes (negative control); "LSU onepot Ficoll 4.5%" is a condition with 4.5% Ficoll 400 added; and "Control Ficoll 4.5%" indicates a condition (negative control) with 4.5% Ficoll 400 added and without using 33 different plasmids each containing one of 33 different LSU r-protein genes. In addition, in Fig. 6, RFU is a relative fluorescent unit.

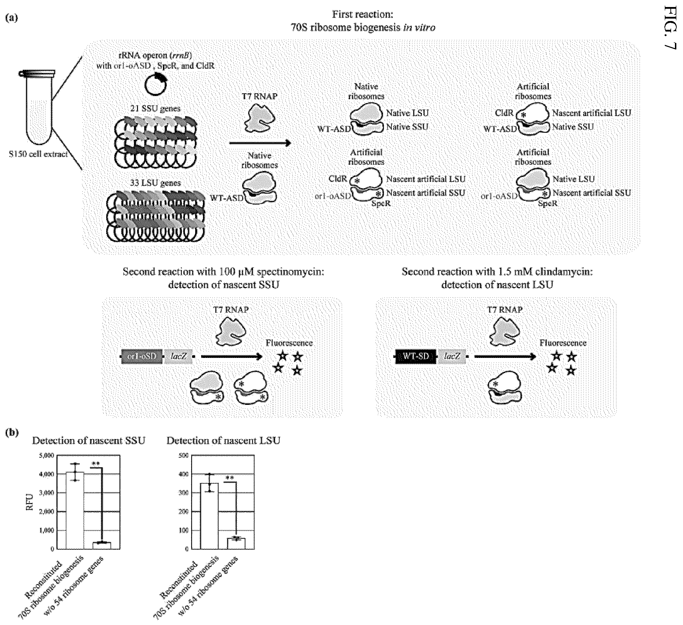

[Fig. 7] Fig. 7 shows the results of simultaneous formation of SSU and LSU *in vitro* by using 54 different plasmids each containing one of 54 different r-protein genes (21 different SSU r-protein genes and 33 different LSU r-protein genes) and a plasmid containing an rRNA operon containing orl-oASD, SpcR, and CldR. Fig. 7a is an experimental scheme for the formation of SSU and LSU *in vitro.* Fig. 7b illustrates data in which the SSU and the LSU were detected. "Reconstituted 70S ribosome biogenesis" is the results of measuring a reporter signal by a bulk assay in the case of the optimized conditions (80 nM native ribosome, 0.01 nM each of the 54 different plasmids containing one of the 54 different r-protein genes, and 0.9 nM of the plasmid containing an rRNA operon with orl-oASD, SpcR, and CldR). An SSU signal was measured in the presence of spectinomycin and an LSU signal was measured in the presence of clindamycin. The "w/o 54 ribosome genes" indicates a condition in which the plasmids containing 54 different r-protein genes were not used. In addition, in Fig. 7, RFU is a relative fluorescence unit, data is a mean ± SD (n = 3), and ** is P < 0.01 (Welch's t-test).

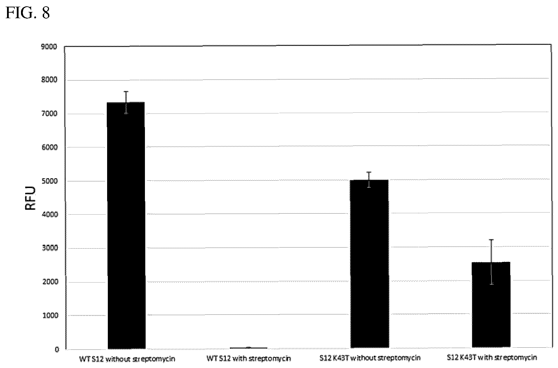

[Fig. 8] Fig. 8 shows the results of performing a bulk assay in the presence of only spectinomycin or spectinomycin and streptomycin in the second reaction while using 54 different plasmids each containing one of 54 different r-protein genes (21 different SSU r-protein genes and 33 different LSU r-protein genes; provided that for the gene encoding 30S r-protein S12, a gene encoding a wild-type S12 or K43T mutant was used) in the first reaction to check the formation of 70S ribosome. In Fig. 8, RFU is a relative fluorescent unit; "WT S12 without streptomycin" is a condition using the gene encoding a wild-type r-protein S12 in the first reaction, and without using streptomycin in the second reaction; "WT S12 with streptomycin" is a condition using the gene encoding a wild-type r-protein S12 in the first reaction, and also using streptomycin in the second reaction; "S12 K43T without streptomycin" is a condition using the gene encoding an r-protein S12 K43T mutant in the first reaction, and without using streptomycin in the second reaction; and "S12 K43T with streptomycin" is a condition using the gene encoding an r-protein S12 K43T mutant in the first reaction, and also using streptomycin in the second reaction. Data is a mean ± SD (n = 3).

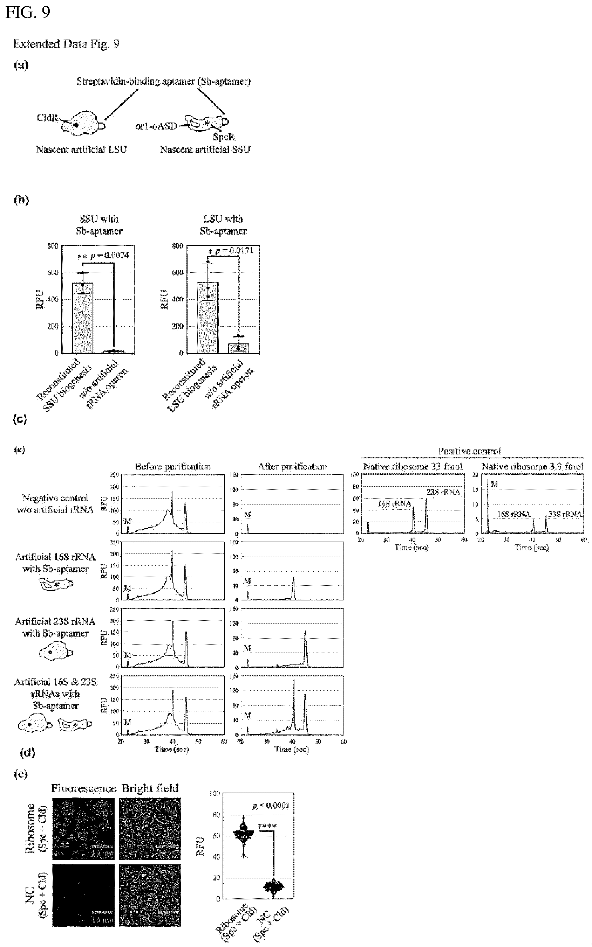

[Fig. 9] Fig. 9a is a schematic diagram of an SSU and an LSU having a streptavidin-binding aptamer (Sb-aptamer). The left graph of Fig. 9b is a graph in which an SSU having an Sb-aptamer was formed *in vitro* using a plasmid encoding 16S rRNA with an Sb-aptamer inserted and 21 different plasmids each containing one of 21 different SSU r-protein genes, and the translational activity of the SSU was detected by a bulk assay. In the left graph of Fig. 9b, "w/o artificial rRNA operon" is a condition without using the 16S rRNA-encoding plasmid. The right graph of Fig. 9b is a graph in which an LSU having an Sb-aptamer was formed *in vitro* using a plasmid encoding 23S rRNA with an Sb-aptamer inserted and 33 plasmids each containing one of 33 different LSU r-protein genes, and the translational activity of the LSU was detected by a bulk assay. In the right graph of Fig. 9b, "w/o artificial rRNA operon" is a condition without using the 23S rRNA-encoding plasmid. Data in Fig. 9b is a mean ± SD (n = 3), * is P < 0.05, ** is P < 0.01 (two-sided Welch's t-test). Fig. 9c shows the results of SSU and/or LSU rRNA electrophoresis analysis before and after purification after performing production of SSU having the 16S rRNA with an Sb-aptamer inserted, performing production of LSU having the 23S rRNA with an Sb-aptamer inserted, and simultaneous production thereof. In Fig. 9c, "Negative control w/o artificial rRNA" is the result of native ribosomes; "Artificial 16S rRNA with Sb-aptamer" is the result when an SSU having the 16S rRNA with an Sb-aptamer inserted was produced; "Artificial 23S rRNA with Sb-aptamer" is the result when an LSU having the 23S rRNA with an Sb-aptamer inserted was produced; and "Artificial 16S & 23S rRNA with Sb-aptamer" is the result when an SSU having the 16S rRNA with an Sb-aptamer inserted and an LSU having the 23S rRNA with an Sb-aptamer inserted were simultaneously produced. Fig. 9d shows the results of a femtoliter droplet assay performed in the presence of spectinomycin and clindamycin on a nascent artificial ribosome purified after simultaneously producing the SSU having an Sb-aptamer and the LSU having an Sb-aptamer. The left images of Fig. 9d are representative photomicrographs of droplets when the femtoliter droplet assay was performed, with a scale bar of 10 μm. In the right graph of Fig. 9d, RFU is a relative fluorescence unit, data is a mean ± SD (n = 3), and *** is P < 0.001 (two-sided Welch's t-test vs. NC(Spc+Cld)). In Fig. 9d, "NC (Spc+Cld)" is the result when using a solution (i.e., foreign substances mixed by purification with streptavidin agarose) obtained by adsorbing native ribosomes onto streptavidin agarose and then eluting the adsorbate.

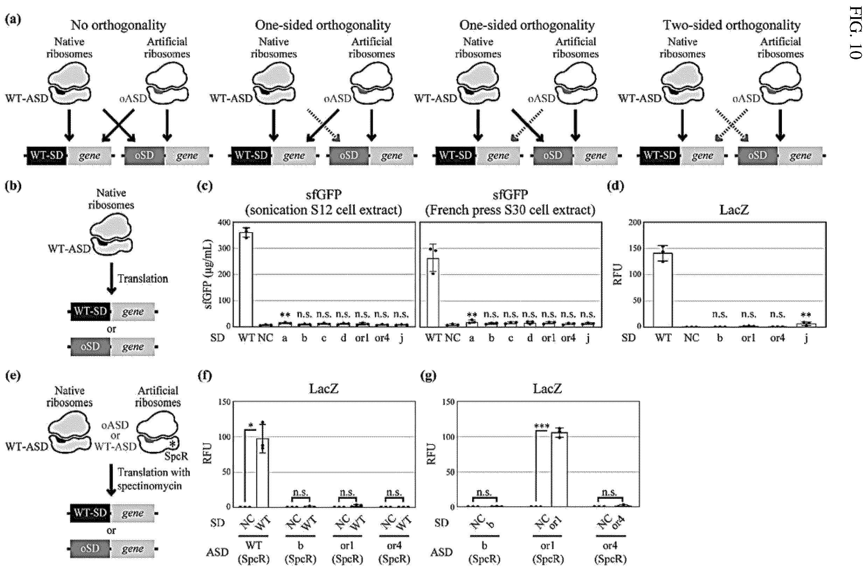

[Fig. 10] Fig. 10a is a schematic diagram of four types of orthogonal translation systems. Fig. 10b is an experimental scheme to select an orthogonal SD (oSD) sequence that does not interact with native ribosomes in *E. coli* cell extract. Fig. 10c shows the results of mixing the pT7_wtSD_sfGFP or pT7_oSD_sfGFP reporter with two kinds of *E. coli* cell extract (sonicated S12 cell extract or French Press S30 cell extract) and measuring a reporter signal. In Fig. 10c, "NC" is a negative control without mixing a reporter, data is a mean ± SD (n = 3), ** is P < 0.01, n.s. is without a significant difference (Dunnett's test vs. NC). Fig. 10d shows the results of mixing the pT7_wtSD_LacZ or pT7_oSD_LacZ reporter with the sonicated S12 cell extract and measuring a reporter signal. In Fig. 10d, RFU is a relative fluorescence unit, "NC" is a negative control without mixing a reporter, data is a mean ± SD (n = 3), ** is P < 0.01, n.s. is without a significant difference (Dunnett's test vs. NC). Fig. 10e is an experimental scheme of screening for an orthogonal anti-SD (oASD)-oSD pair exhibiting two-sided orthogonality in *E. coli* cell extract. Fig. 10f shows the results of mixing spectinomycin and a pT7_wtSD_LacZ reporter with *E. coli* cell extract including ASD- or each oASD (b, or1, or4)- and SpcR-containing artificial ribosomes, and measuring a reporter signal. In Fig. 10f, RFU is a relative fluorescence unit, NC is a negative control without the reporter, data is a mean ± SD (n = 3), * is P < 0.05, n.s. is without a significant difference (Welch's t-test). Fig. 10g shows the results of mixing spectinomycin and a pT7_oSD_LacZ reporter with *E. coli* cell extract including artificial ribosomes containing the corresponding oASD (b, or1, or4) and SpcR, and measuring a reporter signal. In Fig. 10g, RFU is a relative fluorescence unit, NC is a negative control without a reporter, data is a mean ± SD (n = 3), *** is P < 0.01, and n.s. is without a significant difference (Welch's t-test).

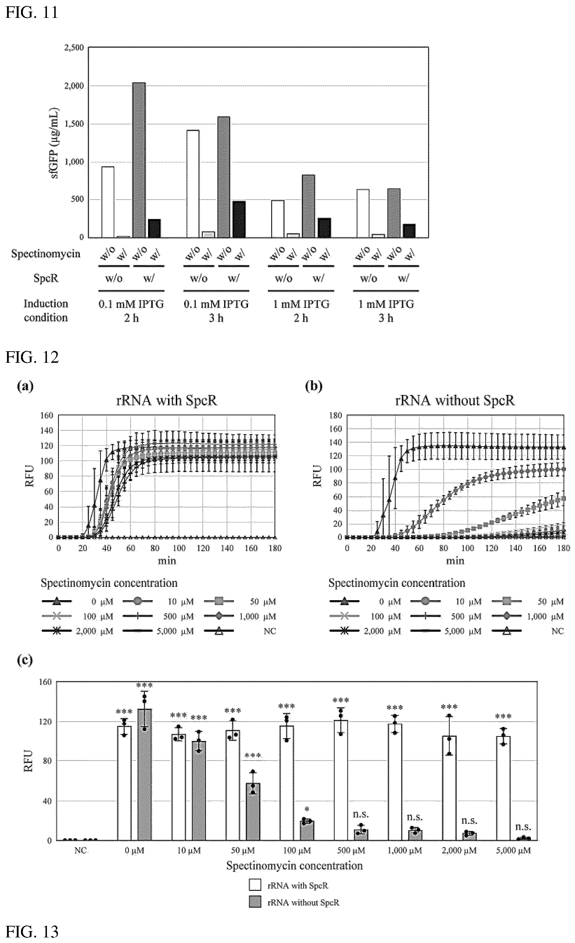

[Fig. 11] Fig. 11 shows the results of optimizing culture conditions for preparing cell extract containing artificial ribosomes. In Fig. 11, w/o represents "without", and w/ represents "with".

[Fig. 12] Fig. 12 shows the results of optimizing a spectinomycin concentration for detecting fluorescence by a LacZ reporter. Figs. 12a and b show the results of determining the fluorescence intensity of the LacZ reporter over time, and Fig. 12c shows the results of fluorescence intensity of the LacZ reporter at each endpoint. In Fig. 12, RFU is a relative fluorescence unit, "NC" is a negative control without the pT7_wtSD_LacZ reporter, data is a mean $\pm$ SD (n = 3), * is P < 0.05, *** is P < 0.001, and n.s. is without a significant difference (Dunnett's test vs. NC).

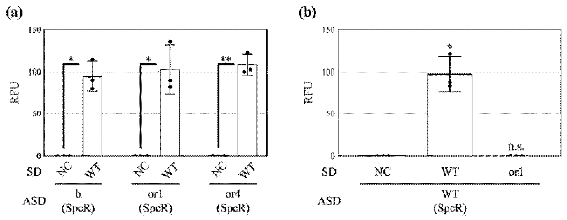

[Fig. 13] Fig. 13a shows the results of checking the transcription and translation activity of each prepared *E. coli* cell extract. In Fig. 13a, RFU is a relative fluorescence unit, NC is a negative control without the pT7_wtSD_LacZ reporter, data is a mean $\pm$ SD (n = 3), * is P < 0.05, and ** is P < 0.01 (Welch's t-test). Fig. 13b shows the results of a control experiment to verify the two-sided orthogonality of a pair of orl-oASD and oSD. In Fig. 13b, RFU is a relative fluorescence unit, "NC" is a negative control without the LacZ reporter, data is a mean $\pm$ SD (n = 3), * is P < 0.05, and n.s. is without a significant difference (Dunnett's test vs. NC).

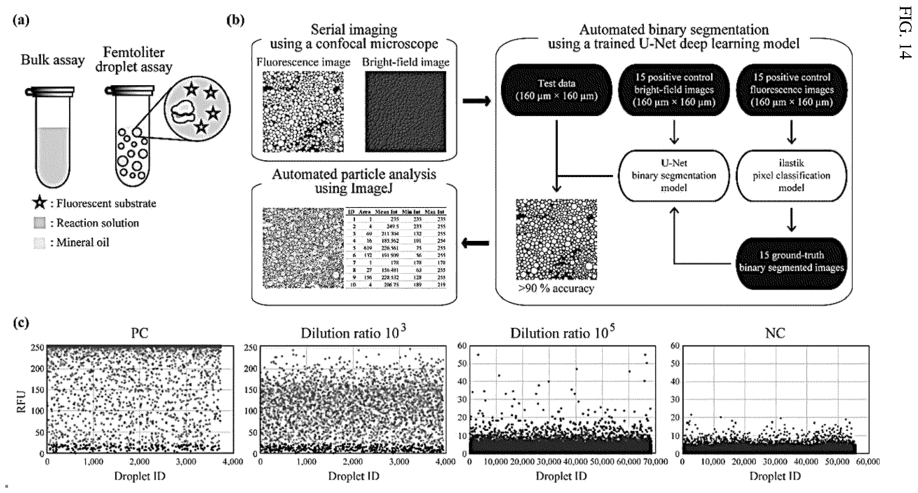

[Fig. 14] Fig. 14a is a schematic diagram in which a conventional bulk assay and a femtoliter droplet assay are compared. Fig. 14b is a schematic diagram of automated analysis of femtoliter droplets by using deep learning. Fig. 14c shows the results of measuring a reporter signal by femtoliter droplet assay in the presence of the pT7_oSD_or1_LacZ reporter and 100 $\mu$M spectinomycin after diluting, $10^3$- or $10^5$-fold, the native ribosomes, the sonicated S12 cell extract containing 4.9 $\mu$M of artificial ribosomes with orl-oASD and SpcR, and the control sonicated S12 cell extract containing only native ribosomes. In Fig. 14c, RFU is a relative fluorescence unit, PC is a positive control using only the sonicated S12 cell extract (undiluted) containing artificial ribosomes with orl-oASD and SpcR, and NC is a negative control using only the sonicated S12 cell extract of the control. In the scatter plots of Fig. 14c, the ordinate represents the average RFU of respective droplets and the abscissa represents ID of each droplet.

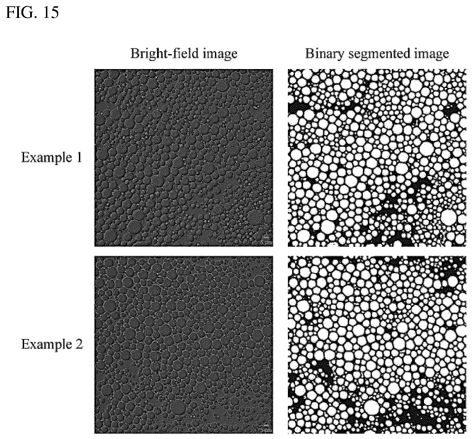

[Fig. 15] in Fig. 15, the left images are bright field images of droplets in the femtoliter droplet assay, and the right images are binarized images (droplets or background) each obtained by converting the bright field image with 90% or more accuracy while using automated analysis of femtoliter droplets by deep learning. In the right images, a white region indicates a droplet, and a black region indicates a background. Fig. 15 shows two examples of droplet images in the femtoliter droplet assay (Examples 1 and 2), and the scale bar is 5 $\mu$m in Fig. 15.

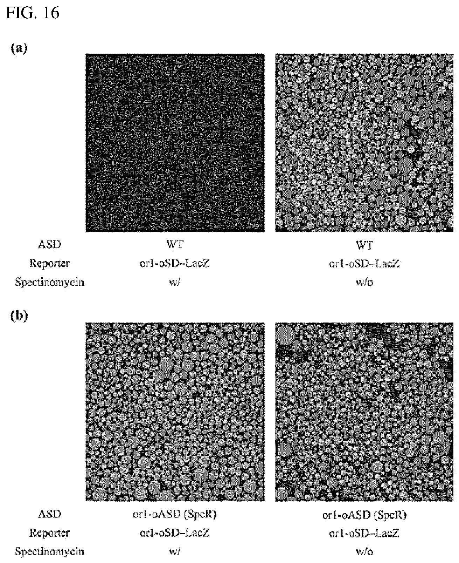

[Fig. 16] Fig. 16a is representative photomicrographs of droplets after the femtoliter droplet assay under conditions where a control sonicated S12 cell extract containing only native ribosomes was mixed with 5 nM pT7_oSD_or1_LacZ reporter and 100 $\mu$M spectinomycin. In Fig. 16a, w/o represents "without", and w/ represents "with". The scale bar is 5 $\mu$m. Fig. 16b is representative photomicrographs of droplets in the femtoliter droplet assay under conditions where a cell extract comprising 4.9 $\mu$M artificial ribosomes with orl-oASD and SpcR was mixed with 5 nM pT7_oSD_or1_LacZ reporter and 100 $\mu$M spectinomycin. In Fig. 16b, w/o represents "without", and w/ represents "with". The scale bar is 5 $\mu$m.

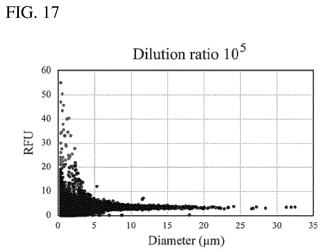

[Fig. 17] Fig. 17 shows the results of creating a scatter plot of the average RFU with respect to the diameter of each droplet by using the data set of Fig. 14c. Description of Embodiments

### 1. Terms

Unless specifically defined otherwise, the terms used herein have the meanings as commonly understood by those skilled in the art of molecular biology, microbiology, organic chemistry, medicine, pharmacy, and so on. When a term defined herein does not have the same meaning as commonly understood, the description herein prevails.

In the present disclosure, the "cell-free protein synthesis system" refers to implementing protein synthesis by performing transcription and translation of a template DNA or translation of a template mRNA *in vitro* without directly using cells such as *E. coli.*

In the present disclosure, the "cell-free synthesis system" refers to performing transcription and translation of a template DNA or translation of a template mRNA *in vitro* to synthesize a polymer without directly using cells such as *E. coli.*

In the present disclosure, a "ribosome" is a molecular complex that reads mRNA genetic information to synthesize a protein. The ribosome is composed of two subunits, a small subunit (SSU) and a large subunit (LSU). For example, in the naturally occurring ribosome of prokaryote *E. coli,* the 70S ribosome usually includes a 30S subunit (SSU) composed of 1 kind of rRNA (16S rRNA) and 21 kinds of r-proteins, and a 50S subunit (LSU) composed of 2 kinds of rRNA (5S rRNA and 23S rRNA) and 33 kinds of r-proteins. In addition, in the naturally occurring ribosome of human, namely a eukaryotic organism, the 80S ribosome usually includes a 40S subunit (SSU) composed of 1 kind of rRNA (18S rRNA) and 33 kinds of r-proteins, and a 60S subunit (LSU) composed of 3 kinds of rRNA (28S rRNA, 5.8S rRNA, and 5S rRNA) and 47 kinds of r-proteins. In addition, in the naturally occurring ribosome of *Pyrococcus furiosus,* which is one of archaea, the 70S ribosome usually includes a 30S subunit (SSU) composed of 1 kind of rRNA (16S rRNA) and 25 kinds of r-proteins, and a 50S subunit (LSU) composed of 2 kinds of rRNA (5S rRNA and 23S rRNA) and 39 kinds of r-proteins.

In the present disclosure, a "ribosomal large subunit protein (LSU r-protein)" refers to a protein that constitutes an LSU, and a "ribosomal small subunit protein (SSU r-protein)" refers to a protein that constitutes an SSU. In addition, in the present disclosure, the term "ribosomal protein (r-protein)" includes any of both an LSU r-protein and an SSU r-protein.

In the present disclosure, the "ribosomal large subunit RNA (LSU rRNA)" refers to rRNA that constitutes an LSU, and the "ribosomal small subunit RNA (SSU rRNA)" refers to rRNA that constitutes an SSU. In addition, in the present disclosure, the term "ribosomal RNA (rRNA)" includes any of both LSU rRNA and SSU rRNA.

In the present disclosure, the wording "assembling LSU r-proteins and LSU rRNAs" refers to complexing LSU r-proteins and LSU rRNAs to assemble a ribosomal LSU, and the wording "assembling SSU r-proteins and an SSU rRNA" refers to complexing SSU r-proteins and an SSU rRNA to assemble a ribosomal SSU.

In the present disclosure, the description regarding the number of r-proteins means the number of kinds of r-protein unless otherwise specified. In addition, in the present disclosure, the description regarding the number of rRNAs means the number of kinds of rRNA unless otherwise specified.

2. Method for producing ribosomal LSU

An embodiment of the present disclosure provides a method for producing a ribosomal LSU in a cell-free protein synthesis system (hereinafter, sometimes referred to as Production Method 1 of the present disclosure). Production Method 1 of the present disclosure is a method for producing a ribosomal LSU, the method comprising the step of:

assembling LSU r-proteins and LSU rRNAs in a cell-free protein synthesis system to form a ribosomal LSU, wherein in the cell-free protein synthesis system, at least one of the LSU r-proteins is generated from a template nucleic acid encoding each corresponding LSU r-protein. Hereinafter, Production Method 1 of the present disclosure will be described in detail.

[Ribosomal LSU]

The ribosomal LSU to be produced in Production Method 1 of the present disclosure may be derived from any of prokaryotes, eukaryotes, and archaea. Examples of the ribosomal LSU to be produced include a ribosomal LSU derived from a prokaryote, preferably a ribosomal LSU derived from *E. coli.*

In the present disclosure, the term "ribosomal LSU derived from a specific biological species" includes not only a naturally occurring ribosomal LSU composed of LSU r-proteins and LSU rRNAs possessed by the specific biological species, but also a variant thereof. For example, the ribosomal LSU derived from *E. coli* includes a naturally occurring ribosomal LSU derived from *E. coli* and a variant thereof. A ribosomal LSU subjected to various modifications in Production Method 1 of the present disclosure may be produced to provide a ribosomal LSU having modified catalytic activity, drug resistance, and the like.

In the present disclosure, examples of the variant of ribosomal LSU include a ribosomal LSU having at least one LSU r-protein having a mutation and/or LSU rRNA having a mutation.

In addition, it has been found that the ribosomal LSU can be formed even when the number of LSU r-proteins constituting the ribosomal LSU is one or more fewer than that of the naturally occurring ribosomal LSU. For example, the naturally occurring ribosome derived from *E. coli* contains 33 different LSU r-proteins (50S r-proteins L1 to L6, L9 to L25, and L27 to L36). However, as shown in the section of Examples described later, the ribosomal LSU can be formed without using all the 33 different LSU r-proteins. Therefore, in Production Method 1 of the present disclosure, it is possible to form a ribosomal LSU that is derived from *E. coli* and is composed of 33 or less different LSU r-proteins. Accordingly, in the present disclosure, examples of the variant of the ribosomal LSU include: a ribosomal LSU in which the number of LSU r-proteins constituting the ribosomal LSU is one or more fewer than that of the naturally occurring ribosomal LSU; or a ribosomal LSU having at least one LSU r-protein having a mutation and/or LSU rRNA having a mutation, and in which the number of LSU r-proteins constituting the ribosomal LSU is one or more fewer than that of the naturally occurring ribosomal LSU.

The number of LSU r-proteins constituting the ribosomal LSU produced by Production Method 1 of the present disclosure is not particularly limited as long as a ribosomal LSU can be formed. For example, when the number of LSU r-proteins constituting the naturally occurring ribosomal LSU is X, the number of LSU r-proteins is X-10 to X, preferably X-5 to X, more preferably X-4 to X, still more preferably X-3 to X, X-2 to X, X-1, or X. Specifically, in Production Method 1 of the present disclosure, a ribosomal LSU derived from *E. coli* may be produced. In this case, of the 33 different LSU r-proteins (50S r-proteins L1 to L6, L9 to L25, and L27 to L36), 23 to 33, preferably 28 to 33, more preferably 29 to 33, still more preferably 30 to 33, 31 to 33, 32, or 33 different LSU r-proteins may be used. In addition, in the ribosomal LSU derived from *E. coli,* the 50S r-protein L21 plays an important role in forming the ribosomal LSU. Therefore, when an *E. coli*-derived ribosomal LSU composed of 32 or less different LSU r-proteins is produced, it is desirable to include at least the 50S r-protein L21 as a constituent LSU r-protein. In addition, 50S r-protein L11 contained in a ribosomal LSU derived from *E. coli* is known to bind thiostrepton to inactivate the ribosomal LSU. Therefore, for example, a ribosomal LSU derived from *E. coli* not containing the 50S r-protein L11 and composed of 32 or less different LSU r-proteins may be produced. This makes it possible to provide a ribosomal LSU having thiostrepton resistance.

In addition, in the ribosomal LSU to be produced in Production Method 1 of the present disclosure, at least one of the LSU rRNAs may be mutated to impart drug resistance. In a case where drug resistance is imparted to LSU rRNA in this manner, when a target protein is produced in the presence of the drug by using a ribosome (nascent ribosome) containing the ribosomal LSU produced by Production Method 1 of the present disclosure, translation by the endogenous ribosome can be suppressed and translation by the nascent ribosome can proceed even in a state where the endogenous ribosome is mixed. For example, clindamycin resistance can be imparted by introducing A2058U mutation into 23S rRNA derived from *E. coli*. Thus, a ribosomal LSU containing A2058U-containing 23S rRNA may be produced by Production Method 1 of the present disclosure. In this case, when a ribosome containing the ribosomal LSU is used to produce a target protein in a cell-free protein synthesis system in the presence of clindamycin, translation by the endogenous ribosome can be suppressed even if the endogenous ribosome is mixed.

[Source of LSU r-proteins used for formation of ribosomal LSU]

In Production Method 1 of the present disclosure, at least one of the LSU r-proteins constituting a ribosomal LSU is supplied from a template nucleic acid encoding each corresponding LSU r-protein. When the number of LSU r-proteins supplied from the template nucleic acids is less than the total number of LSU r-proteins constituting the ribosomal LSU, an LSU r-protein(s) (in a protein form) is used in addition to the template nucleic acids as a source of the LSU r-proteins.

That is, all of the LSU r-proteins contained in the ribosomal LSU may be composed of the LSU r-proteins generated from the template nucleic acids. In this case, only the template nucleic acids may be added to the cell-free protein synthesis system as a source of the LSU r-proteins, and the LSU r-protein(s) (in a protein form) may not be added.

Further, part of the LSU r-proteins contained in the ribosomal LSU may be composed of the LSU r-proteins generated from the template nucleic acids. In this case, the rest LSU r-proteins contained in the ribosomal LSU are composed of r-proteins supplied in a protein form. That is, in this case, the LSU r-protein(s) (in a protein form) may be added, as a source of the LSU r-proteins, to the cell-free protein synthesis system together with the template nucleic acids.

In Production Method 1 of the present disclosure, at least one of the LSU r-proteins constituting the ribosomal LSU may be supplied from the template nucleic acid(s). However, the number of LSU r-proteins supplied from the template nucleic acids (the number of template nucleic acids used) among the LSU r-proteins constituting the ribosomal LSU is, for example, 2 or more, 5 or more, 10 or more, 15 or more, 20 or more, 25 or more, or 30 or more. As a preferred embodiment of Production Method 1 of the present disclosure, all of the LSU r-proteins constituting the ribosomal LSU are supplied from the template nucleic acids. Further, for example, a ribosomal LSU derived from *E. coli* may be produced. In this case, among the LSU r-proteins constituting the ribosomal LSU, the number of LSU r-proteins supplied from the template nucleic acids (the number of template nucleic acids used) is 1 or more, 2 or more, 5 or more, 10 or more, 15 or more, 20 or more, 25 or more, 26 or more, 27 or more, 28 or more, 29 or more, 30 or more, 31 or more, or 32 or more. In a preferred embodiment of producing a ribosomal LSU derived from *E. coli,* all of the LSU r-proteins constituting the ribosomal LSU are supplied from the template nucleic acids.

The following describes a template nucleic acid(s) encoding the LSU r-protein(s) used as a source of the LSU r-proteins constituting a ribosomal LSU and an LSU r-protein(s) (in a protein form) optionally used as the source.

· Template nucleic acid encoding LSU r-protein

The template nucleic acid encoding an LSU r-protein may be either DNA or mRNA as long as the LSU r-protein can be generated in a cell-free protein synthesis system. When the template nucleic acid is DNA, it is transcribed and translated in a cell-free protein synthesis system to supply the LSU r-protein. When the template nucleic acid is mRNA, it is translated in a cell-free protein synthesis system to supply the LSU r-protein.

The template DNA encoding an LSU r-protein should be operably linked by a promoter to the cDNA encoding an LSU r-protein. The promoter may be any promoter as long as it is compatible with RNA polymerase used in the cell-free protein synthesis system, and examples thereof include a T7 promoter, an SP6 promoter, a T3 promoter, various promoters that are endogenously used by cells, or an artificially designed promoter. In addition, the template DNA encoding an LSU r-protein may include a terminator, poly-A, 3'UTR, and the like on the 3' downstream side of the cDNA encoding an LSU r-protein. In addition, the template DNA encoding an LSU r-protein may include an enhancer on the 3' downstream side or 5' upstream side of the cDNA encoding an LSU r-protein. In addition, in the case of the cell-free protein synthesis system, a Shine-Dalgarno (SD) sequence may be included between the promoter and the cDNA encoding an LSU r-protein. In addition, the template DNA encoding an LSU r-protein may include an operator in the vicinity of the promoter in order to control expression.

In addition, when two or more of the LSU r-proteins constituting a ribosomal LSU are supplied from each template DNA encoding the corresponding LSU r-protein, the template nucleic acid may encode one LSU r-protein per molecule, or may encode two or more LSU r-proteins per molecule.

The template DNA encoding an LSU r-protein may be double-stranded DNA, and may be either linear or circular such as a plasmid.

The template mRNA encoding an LSU r-protein may include a coding region (CDS) encoding the LSU r-protein, and a 5' untranslated region (5' UTR) may be linked to the 5' terminal side thereof and a 3' untranslated region (3' UTR) may be linked to the 3' terminal side thereof. In the template mRNA encoding an LSU r-protein, a regulatory sequence such as a cap or an SD sequence may be added to the 5' terminal side of the 5' UTR, and a terminator or a polyA tail may be added to the 3' terminal side of the 3' UTR.

The nucleotide sequence of cDNA and CDS encoding each LSU r-protein is known. Examples of each cDNA encoding an LSU r-protein derived from E. coli include 50S r-protein L1: Gene ID: 948483, 50S r-protein L2: Gene ID: 947820, 50S r-protein L3 : Gene ID: 947817, 50S r-protein L4: Gene ID: 947818, 50S r-protein L5: Gene ID: 947800, 50S r-protein L6: Gene ID: 947803, 50S r-protein L9: Gene ID: 948720, 50S r-protein L10: Gene ID: 948490, 50S r-protein L11: Gene ID: 948484, 50S r-protein L12: Gene ID: 948489, 50S r-protein L13: Gene ID: 947828, 50S r-protein L14: Gene ID: 947809, 50S r-protein L15: Gene ID: 947798, 50S r-protein L16: Gene ID: 947806, 50S r-protein L17: Gene ID: 947784, 50S r-protein L18: Gene ID: 947804, 50S r-protein L19: Gene ID: 947096, 50S r-protein L20: Gene ID: 945152, 50S r-protein L21: Gene ID: 949057, 50S r-protein L22: Gene ID: 947813, 50S r-protein L23: Gene ID: 947819, 50S r-protein L24: Gene ID: 947810, 50S r-protein L25: Gene ID: 945618, 50S r-protein L27: Gene ID: 945190, 50S r-protein L28: Gene ID: 946941, 50S r-protein L29: Gene ID: 947807, 50S r-protein L30: Gene ID: 947797, 50S r-protein L31: Gene ID: 948425, 50S r-protein L32 :Gene ID: 945657, 50S r-protein L33: Gene ID: 946318, 50S r-protein L34: Gene ID: 948216, 50S r-protein L35: Gene ID: 946349, or 50S r-protein L36: Gene ID: 947805. Each is registered in a database provided by NIH (National Institute of Health).

In Production Method 1 of the present disclosure, when a ribosomal LSU containing a r-protein with a mutation introduced is produced, a desired mutation may be introduced into a template nucleic acid encoding the LSU r-protein.

The template nucleic acid encoding an LSU r-protein may be prepared by a known genetic engineering technique.

· LSU r-protein (in protein form) optionally used

Meanwhile, in Production Method 1 of the present disclosure, the LSU r-protein(s) (in a protein form) optionally used as a source of the LSU r-proteins constituting the ribosomal LSU may be separated from a naturally occurring ribosome, or may be produced using a genetic engineering technique.

[Source of LSU r-RNAs used for formation of ribosomal LSU]

In Production Method 1 of the present disclosure, the source of LSU rRNAs constituting a ribosomal LSU is an LSU rRNA(s) (in an RNA form) and/or a template DNA(s) encoding an LSU rRNA(s). When the LSU rRNA(s) (in an RNA form) is used as a source of LSU rRNAs, the LSU rRNA(s) is directly assembled into the ribosomal LSU in a cell-free protein synthesis system. In addition, when the template DNA(s) encoding an LSU rRNA is used as a source of LSU rRNAs, the LSU rRNA(s) transcribed from the template DNA(s) in a cell-free protein synthesis system encoding an LSU rRNA is directly assembled into the ribosomal LSU.

For example, in the case of producing a ribosomal LSU derived from E. coli, as a source of LSU rRNAs, 5S rRNA or a template DNA encoding 5S rRNA and 23S rRNA or a template DNA encoding 23S rRNA are used.

In Production Method 1 of the present disclosure, preferable examples of the source of LSU rRNAs constituting a ribosomal LSU include a template DNA(s) encoding an LSU rRNA. A mutation can be easily introduced into the template DNA. Thus, when a template DNA encoding an LSU rRNA is used, a ribosomal LSU containing the LSU rRNA with a mutation introduced can be easily produced.

The template DNA encoding an LSU rRNA should be operably linked by a promoter to the cDNA encoding an LSU rRNA. The promoter may be any promoter as long as it is compatible with RNA polymerase used in the cell-free protein synthesis system, and examples thereof include a T7 promoter, an SP6 promoter, a T3 promoter, various promoters that are endogenously used by cells, or an artificially designed promoter. In addition, the template DNA encoding an LSU rRNA may include a terminator and the like on the 3' downstream side of the cDNA encoding an LSU rRNA. In addition, the template DNA encoding an LSU rRNA may include an operator in the vicinity of the promoter in order to control expression.

Also, a ribosomal LSU derived from E. coli may be produced. In this case, it is also possible to use, as the template DNA encoding an LSU rRNA, a template DNA encoding the E. coli 5S rRNA and a template DNA encoding the 23S rRNA gene, or rrnB (operon encoding 16S rRNA, 23S rRNA, and 5S rRNA), which is an E. coli rRNA operon, or the like.

The nucleotide sequence of DNA encoding each LSU rRNA is known. For example, in the case of LSU rRNA derived from E. coli, DNA encoding 5S rRNA is registered as Gene ID: 948471, and DNA encoding 23S rRNA is registered as Gene ID: 948473 in a database provided by NIH.

The template DNA encoding an LSU rRNA may be double-stranded DNA, and may be either linear or circular such as a plasmid. The template DNA encoding an LSU rRNA may be prepared by a known genetic engineering technique.

In Production Method 1 of the present disclosure, when a ribosomal LSU containing a mutated LSU rRNA is produced, a desired mutation may be introduced into an LSU rRNA (in an RNA form) and/or a template DNA encoding an LSU rRNA used as a source.

[Formation of ribosomal LSU in cell-free protein synthesis system]

In Production Method 1 of the present disclosure, a source of LSU r-proteins (template nucleic acids encoding LSU r-proteins and optionally LSU r-protein(s) (in a protein form)) and a source of LSU rRNAs (LSU rRNA(s) (in an RNA form) and/or a template DNA(s) encoding an LSU rRNA(s)) may be incubated in a reaction solution of a cell-free protein synthesis system, and LSU r-proteins and LSU rRNAs are then assembled to form a ribosomal LSU.

The scheme of ribosomal LSU formation in Production Method 1 of the present disclosure is as follows. When the template nucleic acid encoding an LSU r-protein used as a source of LSU r-proteins is incubated in a reaction solution of a cell-free protein synthesis system, the template nucleic acid is transcribed/translated or translated to generate the LSU r-protein. In addition, when a template DNA encoding an LSU rRNA is used as a source of LSU rRNAs, the template DNA is incubated in a reaction solution of a cell-free protein synthesis system and is transcribed to generate the LSU rRNA. Then, in the reaction solution of the cell-free protein synthesis system, (i) LSU r-proteins generated from template nucleic acids encoding the LSU r-proteins; (ii) an LSU r-protein(s) added in a protein form as a source of the LSU r-proteins, if necessary (when part of the LSU r-proteins contained in the ribosomal LSU is composed of the LSU r-proteins generated from the template nucleic acids); and (iii) LSU rRNA(s) (added in an RNA form) and/or LSU rRNA(s) generated from template DNA(s) encoding LSU rRNA(s) assemble to form a ribosomal LSU.

In Production Method 1 of the present disclosure, as the cell-free protein synthesis system, a cell-free protein synthesis system using a cell extract may be used, or a reconstituted cell-free protein synthesis system (PURE System) consisting of factors in which elements necessary for transcription/translation or transcription are independently purified may be used. In the cell-free protein synthesis system using the cell extract, for example, a cell extract such as *E. coli,* yeast, wheat germ, or rabbit reticulocyte extract can be used as the reaction solution.

The concentration of each template nucleic acid encoding an LSU r-protein in the reaction solution of the cell-free protein synthesis system may be appropriately adjusted to a range in which the ribosomal LSU can be formed according to the type and concentration of the source of the LSU rRNAs contained in the reaction solution, the concentration of the ribosome used for translation in the cell-free protein synthesis system, and the like. However, from the viewpoint of efficiently forming the ribosomal LSU, the concentration of each template nucleic acid encoding an LSU r-protein is from 1 fM to 1 $\mu$M, preferably from 1 pM to 100 pM, and more preferably from 5 pM to 10 pM.

The LSU r-protein(s) (in a protein form) as a source of the LSU r-proteins may be used (part of the LSU r-proteins contained in the ribosomal LSU may be composed of the LSU r-proteins generated from the template nucleic acids). In this case, the concentration of each LSU r-protein added to the reaction solution of the cell-free protein synthesis system may be appropriately adjusted to a range in which the ribosomal LSU can be formed. For example, the concentration of each LSU r-protein added in the form of a protein is from 1 pM to 1 M, preferably from 1 nM to 1 mM, and more preferably from 10 nM to 1 $\mu$M.

The LSU rRNAs (in an RNA form) may be used as a source of LSU rRNAs in a cell-free protein synthesis system. In this case, the concentration of each LSU rRNA added to the reaction solution of the cell-free protein synthesis system may be appropriately adjusted to a range in which the ribosomal LSU can be formed. For example, the concentration of each LSU rRNA added in the form of an RNA is from 1 pM to 1 M, preferably from 1 nM to 1 mM, and more preferably from 10 nM to 1 $\mu$M.

The template DNA encoding an LSU rRNA may be used as a source of LSU rRNAs in a cell-free protein synthesis system. In this case, the concentration of the template DNA added to the reaction solution of the cell-free protein synthesis system may be appropriately adjusted to a range in which the ribosomal LSU can be formed. However, from the viewpoint of efficiently forming the ribosomal LSU, the concentration of the template DNA is from 1 fM to 1 $\mu$M, preferably from 1 pM to 10 nM, and more preferably from 200 pM to 1.5 nM.

Further, DNA may be used as a template nucleic acid encoding an LSU r-protein and a template DNA encoding an LSU rRNA may be used as a source of LSU rRNAs. In this case, the reaction solution of the cell-free protein synthesis system includes a DNA-dependent RNA polymerase for transcribing these DNAs. As the DNA-dependent RNA polymerase, one compatible with the promoter of the template DNA may be used. For example, T7 RNA polymerase may be used when a T7 promoter is used in the template DNA; T3 RNA polymerase may be used when a T3 promoter is used in the template DNA; SP6 RNA polymerase may be used when a SP6 promoter is used in the template DNA; natural RNA polymerase (e.g., *E.coli* RNA polymerase) may be used when various promoters endogenously used by the cell are used; and when an artificially designed promoter is used, any RNA polymerase capable of transcribing the promoter may be used. A cell-free protein synthesis system using a cell extract may be used. In this case, if the cell extract contains a DNA-dependent RNA polymerase compatible with the promoter of the template DNA, the DNA-dependent RNA polymerase may not be separately added. In addition, in the case of a cell-free protein synthesis system using a cell extract, the cell extract may be free of or may not contain a sufficient amount of a DNA-dependent RNA polymerase compatible with the promoter of the template DNA. In this case, a necessary amount of DNA-dependent RNA polymerase may be added. The concentration of the DNA-dependent RNA polymerase in the reaction solution of the cell-free protein synthesis system may be appropriately set within a range in which the template DNA can be transcribed.

Further, the reaction solution of the cell-free protein synthesis system includes ribosomes (hereinafter, endogenous ribosomes) in order to translate mRNA transcribed from the template DNA encoding an LSU r-protein. In the case of a cell-free protein synthesis system using a cell extract, the cell extract may contain endogenous ribosomes capable of translating mRNA transcribed from the template DNA encoding an LSU r-protein at an appropriate concentration. In this case, no additional endogenous ribosomes may be added. In addition, in the case of a cell-free protein synthesis system using a cell extract, the cell extract may be free of or may not contain a sufficient amount of endogenous ribosomes. In this case, a necessary amount of endogenous ribosomes may be added. The added ribosomes may be either natural or modified as long as it can translate the LSU r-protein. The concentration of ribosomes in the reaction solution of the cell-free protein synthesis system may be appropriately adjusted to a range in which the ribosomal LSU can be formed according to the concentration of the template nucleic acid encoding an LSU r-protein contained in the reaction solution, and the like. However, from the viewpoint of efficiently forming the ribosomal LSU, the concentration is from 1 pM to 1 M, preferably from 1 nM to 1 mM, more preferably from 10 nM to 1 $\mu$M, and still more preferably from 50 nM to 500 nM.

The reaction solution of the cell-free protein synthesis system contains components such as NTPs (ATP, GTP, UTP, and CTP), tRNAs, amino acids (20 kinds), and ATP regeneration factors (e.g., a combination of phosphoenolpyruvate and pyruvate kinase, a combination of creatine phosphate and creatine kinase) for transcription, translation, ATP regeneration, and the like. Furthermore, in addition to the above-described components, the reaction solution of the cell-free protein synthesis system may contain coenzymes (e.g., NAD, NADH, NADP, NADPH, FAD, FADH, CoA), salts (e.g., potassium glutamate, ammonium glutamate, magnesium glutamate, potassium oxalate), folates (e.g., folinic acid), polyamines (e.g., spermidine, putrescine), buffers, reducing agents (e.g., DTT, TCEP, CTBA, BME), crowding agents (e.g., Ficoll 70 and 400, polyethylene glycol 3350, 6000, 8000), antibacterial agents, chaperones (e.g., GroEL/ES, trigger factor, DnaKJE), other known factors known to support assembly of ribosomes, and osmotic pressure regulators (e.g., TMAO, urea). In the case of a cell-free protein synthesis system using a cell extract, these components may be contained in a sufficient amount in the cell extract. In this case, no additional components may be added. In addition, in the case of a cell-free protein synthesis system using a cell extract, the cell extract may be free of these components or may not contain a sufficient amount of these components. In this case, these components may be added in necessary amounts. In the reaction solution of the cell-free protein synthesis system, the concentrations of these components can be appropriately selected within a range that can be usually used in the cell-free protein synthesis system. In addition, a continuous dialysis method may be used to improve the reaction efficiency.

The reaction solution of the cell-free protein synthesis system may contain a component that promotes the formation of ribosomal LSU. Examples of such a component include polysucrose. Polysucrose is a side-chain-rich, neutral, hydrophilic synthetic polysaccharide made from sucrose and is a substance identified as CAS No. 26873-85-8. The molecular weight of polysucrose is not particularly limited, and is, for example, from 10,000 to 5,000,000, preferably from 50,000 to 1,000,000, and more preferably from 300,000 to 600,000. Polysucrose is commercially available as "Ficoll® 400" (Sigma-Aldrich), "Ficoll® PM 400" (GE Healthcare), "Ficoll® PM 70" (GE Healthcare), "Polysucrose 400" (FUJIFILM Wako Pure Chemical), and the like, and these commercially available products may be used. When polysucrose is contained in the reaction solution of the cell-free protein synthesis system, the concentration of polysucrose in the reaction solution of the cell-free protein synthesis system is, for example, from 0.1 to 20 wt/v%, preferably from 0.5 to 10 wt/v%, and more preferably from 1 to 6 wt/v%.

The time for incubating the reaction solution of the cell-free protein synthesis system may be a time required for generation of the LSU r-proteins from the template nucleic acids encoding the LSU r-proteins, generation of the SSU rRNA from the template DNA when the template DNA encoding the SSU rRNA is used as the source of the SSU rRNA, and assembly of the ribosomal LSU. However, the time is, for instance, about 1 to 72 hours, preferably about 2 to 48 hours, and more preferably about 3 to 12 hours.

The temperature for incubating the reaction solution of the cell-free protein synthesis system may be a temperature allowing for generation of the LSU r-proteins from the template nucleic acids encoding the LSU r-proteins, generation of the LSU rRNAs from the template DNAs when the template DNAs encoding the LSU rRNAs are used as the source of the LSU rRNAs, and assembly of the ribosomal LSU. However, the temperature is, for example, about 5 to 50°C, preferably about 15 to 40°C, and more preferably about 25 to 37°C.

In the reaction solution after incubation, the generated ribosomal LSU is present alone. Alternatively, in a case where the origin is the same as or a closely related species with the endogenous ribosome, at least part of the generated ribosomal LSU is present in association with the endogenous ribosomal SSU to form a ribosome. The generated ribosomal LSU (including a state in which a ribosome associated with the endogenous ribosomal SSU is formed) can be purified by a known method such as centrifugation, chromatography, beads, or electrophoresis. In addition, a tag may be added to a template nucleic acid encoding an LSU r-protein, and when a template DNA encoding an LSU rRNA is used as a source of the LSU rRNAs, a tag may be added to the template DNA. This also enables affinity purification. Examples of the tag include an MS2 tag, a FLAG tag, a His tag, a Strep tag, or a GST tag.

[Production of polymer using ribosomal LSU produced by Production Method 1 of the present disclosure]

The ribosomal LSU produced by Production Method 1 of the present disclosure may be used for producing a polymer in a cell-free synthesis system by forming a ribosome with a ribosomal SSU derived from the same species or a related species. In the production of a polymer in a cell-free synthesis system, the polymer may be synthesized from a template DNA or mRNA encoding a target polymer in a cell-free synthesis system using a ribosome including the ribosomal LSU produced by Production Method 1 of the present disclosure. The cell-free synthesis system may have components capable of transcribing and translating the template DNA or translating the template mRNA depending on the type of the polymer to be produced and the like. An example of the cell-free synthesis system includes a system having the same composition as that of the cell-free protein synthesis system.

The ribosome including the ribosomal LSU produced by Production Method 1 of the present disclosure can be used, for example, for synthesis of a peptide or protein using naturally occurring amino acids as a monomer.

In addition, in Production Method 1 of the present disclosure, a mutation can be easily introduced into an LSU r-protein and/or an LSU rRNA, and a ribosomal LSU having various catalytic activities can be produced. Therefore, the ribosome including the ribosomal LSU produced by Production Method 1 of the present disclosure may also be used for synthesis of a peptide or protein containing a non-natural amino acid, for example, while using a non-natural amino acid as at least one of monomers, depending on its catalytic activity. In addition, the ribosome including the ribosomal LSU produced by Production Method 1 of the present disclosure may also be used for synthesis of a non-natural polymer linked by an ester bond or a thioester bond while using, for example, a non-natural monomer that can be linked by an ester bond or a thioester bond, depending on its catalytic activity.

Further, a ribosomal SSU derived from the same species or a similar species may be added as necessary to the reaction solution of the cell-free protein synthesis system in which the ribosomal LSU is generated by performing Production Method 1 of the present disclosure. Furthermore, the polymer may also be produced by adding and incubating a template DNA or mRNA encoding the polymer and, if necessary, a monomer as a raw material.

3. Method for producing ribosomal SSU

Another embodiment of the present disclosure provides a method for producing a ribosomal SSU in a cell-free protein synthesis system (hereinafter, sometimes referred to as Production Method 2 of the present disclosure). Production Method 2 of the present disclosure is a method for producing a ribosomal SSU, the method comprising the step of:

assembling SSU r-proteins and an SSU rRNA in a cell-free protein synthesis system to form a ribosomal SSU, wherein in the cell-free protein synthesis system, at least two of the SSU r-proteins are generated from template nucleic acids encoding the corresponding SSU r-proteins. Hereinafter, Production Method 2 of the present disclosure will be described in detail.

[Ribosomal SSU]

The ribosomal SSU to be produced in Production Method 2 of the present disclosure may be derived from any of prokaryotes, eukaryotes, and archaea. Examples of the ribosomal SSU to be produced include a ribosomal SSU derived from a prokaryote, preferably a ribosomal SSU derived from *E. coli.*

In the present disclosure, the term "ribosomal SSU derived from a specific biological species" includes not only a naturally occurring ribosomal SSU composed of SSU r-proteins and an SSU rRNA possessed by the specific biological species, but also a variant thereof. For example, the ribosomal SSU derived from *E. coli* includes a naturally occurring ribosomal SSU derived from *E. coli* and a variant thereof. A ribosomal SSU subjected to various modifications in Production Method 2 of the present disclosure may be produced to provide a ribosomal SSU having modified catalytic activity, drug resistance, and the like.

In the present disclosure, examples of the variant of ribosomal SSU include a ribosomal SSU having at least one SSU r-protein having a mutation and/or SSU rRNA having a mutation.

In addition, it has been found that the ribosomal SSU can be formed even when the number of SSU r-proteins constituting the ribosomal SSU is one or more fewer than that of the naturally occurring ribosomal SSU. For example, the naturally occurring ribosome derived from *E. coli* contains 21 different SSU r-proteins (30S r-proteins S1 to S21). However, as shown in the section of Examples described later, the ribosomal SSU can be formed without using all the 21 different SSU r-proteins. Therefore, in Production Method 2 of the present disclosure, it is possible to form a ribosomal SSU that is derived from *E. coli* and is composed of 21 or less different SSU r-proteins. Accordingly, in the present disclosure, examples of the variant of the ribosomal SSU include: a ribosomal SSU in which the number of SSU r-proteins constituting the ribosomal SSU is one or more fewer than that of the naturally occurring ribosomal SSU; or a ribosomal SSU having at least one SSU r-protein having a mutation and/or SSU rRNA having a mutation, and in which the number of SSU r-proteins constituting the ribosomal SSU is one or more fewer than that of the naturally occurring ribosomal SSU.

The number of SSU r-proteins constituting the ribosomal SSU produced by Production Method 2 of the present disclosure is not particularly limited as long as a ribosomal SSU can be formed. For example, when the number of SSU r-proteins constituting the naturally occurring ribosomal SSU is Y, the number of SSU r-proteins is Y-10 to Y, preferably Y-5 to Y, more preferably Y-4 to Y, and still more preferably Y-3 to Y, Y-2 to Y, Y-1, or Y. Specifically, in Production Method 2 of the present disclosure, a ribosomal SSU derived from *E. coli* may be produced. In this case, of the 21 different SSU r-proteins (30S r-proteins S1 to S21), 11 to 21, preferably 16 to 21, more preferably 17 to 21, and still more preferably 18 to 21, 19 to 21, 20, or 21 different SSU r-proteins may be used. In addition, in the ribosomal SSU derived from *E. coli,* the 30S r-proteins S12 and S16 play an important role in forming the ribosomal SSU. Therefore, when an *E. coli*-derived ribosomal SSU composed of 20 or less different SSU r-proteins is produced, it is desirable to include at least the 30S r-proteins S12 and S16 as constituent SSU r-proteins.

In addition, in the ribosomal SSU to be produced in Production Method 2 of the present disclosure, the anti-Shine-Dalgarno (ASD) sequence of the SSU rRNA may be set so as to be able to recognize a Shine-Dalgarno sequence (SD) that is not recognized by an ASD sequence of an SSU rRNA of a ribosome (endogenous ribosome) used for translation in the cell-free protein synthesis system in Production Method 2 of the present disclosure. Thus, the ribosome (hereinafter, nascent ribosome) including the ribosomal SSU produced by Production method 2 of the present disclosure and the endogenous ribosome are designed to recognize a different SD from each other. In this case, when a target polymer is produced using a nascent ribosome, one having an SD sequence that can be recognized by the nascent ribosome is used as a template DNA or a template mRNA encoding the target polymer. This makes it possible to suppress translation by the endogenous ribosomes and more specifically detect translation by the nascent ribosomes even in a state in which the endogenous ribosomes are mixed.

In addition, in the ribosomal SSU to be produced in Production Method 2 of the present disclosure, the SSU rRNA may be mutated to impart drug resistance. In a case where drug resistance is imparted to SSU rRNA in this manner, when a target protein is produced in the presence of the drug by using a ribosome (nascent ribosome) containing the ribosomal SSU produced by Production Method 2 of the present disclosure, translation by the endogenous ribosome can be suppressed and translation by the nascent ribosome can proceed even in a state where the endogenous ribosome is mixed. For example, spectinomycin resistance can be imparted by introducing C1192U mutation into 16S rRNA derived from *E. coli.* Thus, a ribosomal SSU including C1192U-containing 16S rRNA may be produced by Production Method 2 of the present disclosure. In this case, when a ribosome containing the ribosomal SSU is used to produce a target polymer in a cell-free synthesis system in the presence of spectinomycin, translation by the endogenous ribosome can be suppressed even if the endogenous ribosome is mixed.

Further, in the ribosomal SSU to be produced in Production Method 2 of the present disclosure, at least one of the SSU r-proteins may be mutated to impart drug resistance. In a case where drug resistance is imparted to an SSU r-protein in this manner, when a target polymer is produced in the presence of the drug by using a ribosome (nascent ribosome) containing the ribosomal SSU produced by Production Method 2 of the present disclosure, translation by the endogenous ribosome can be suppressed and translation by the nascent ribosome can proceed even in a state where the endogenous ribosome is mixed. For example, streptomycin resistance can be imparted by introducing K43T mutation into 30S r-protein S12 derived from *E. coli.* Thus, a ribosomal SSU including K43T-containing 30S r-protein S12 may be produced by Production Method 2 of the present disclosure. In this case, when a ribosome containing the ribosomal SSU is used to produce a target polymer in a cell-free synthesis system in the presence of streptomycin, translation by the endogenous ribosome can be suppressed even if the endogenous ribosome is mixed.

[Source of SSU r-proteins used for formation of ribosomal SSU]

In Production Method 2 of the present disclosure, at least two of the SSU r-proteins constituting a ribosomal SSU are each supplied from a template nucleic acid encoding the SSU r-protein. When the number of SSU r-proteins supplied from the template nucleic acids is less than the total number of SSU r-proteins constituting the ribosomal SSU, an SSU r-protein(s) (in a protein form) is used in addition to the template nucleic acids as a source of the SSU r-proteins.

That is, all of the SSU r-proteins contained in the ribosomal SSU may be composed of the SSU r-proteins generated from the template nucleic acids. In this case, only the template nucleic acids may be added to the cell-free protein synthesis system as a source of the SSU r-proteins, and the SSU r-protein(s) (in a protein form) may not be added.

Further, part of the SSU r-proteins contained in the ribosomal SSU may be composed of the SSU r-proteins generated from the template nucleic acids. In this case, the rest SSU r-proteins contained in the ribosomal SSU are composed of r-proteins supplied in a protein form. That is, in this case, the SSU r-proteins (in a protein form) may be added, as a source of the SSU r-protein, to the cell-free protein synthesis system together with the template nucleic acids.

In Production Method 2 of the present disclosure, at least two of the SSU r-proteins constituting the ribosomal SSU may be supplied from the template nucleic acids. However, the number of SSU r-proteins supplied from the template nucleic acids (the number of template nucleic acids used) among the SSU r-proteins constituting the ribosomal SSU is, for example, 3 or more, 5 or more, 10 or more, 15 or more, or 20 or more. As a preferred embodiment of Production Method 2 of the present disclosure, all of the SSU r-proteins constituting the ribosomal SSU are supplied from the template nucleic acids. Further, for example, a ribosomal SSU derived from *E. coli* may be produced. In this case, the number of SSU r-proteins supplied from the template nucleic acids (the number of template nucleic acids used) among the SSU r-proteins constituting the ribosomal SSU is, 2 or more, 3 or more, 5 or more, 10 or more, 15 or more, or 20 or more. In a preferred embodiment of producing a ribosomal SSU derived from *E. coli,* all of the SSU r-proteins constituting the ribosomal SSU are supplied from the template nucleic acids.

The following describes a template nucleic acid(s) encoding the SSU r-protein(s) used as a source of the SSU r-proteins constituting a ribosomal SSU and an SSU r-protein(s) (in a protein form) optionally used as the source.

· Template nucleic acid encoding SSU r-protein

The template nucleic acid encoding an SSU r-protein may be either DNA or mRNA as long as the SSU r-protein can be generated in a cell-free protein synthesis system. When the template nucleic acid is DNA, it is transcribed and translated in a cell-free protein synthesis system to supply the SSU r-protein. When the template nucleic acid is mRNA, it is translated in a cell-free protein synthesis system to supply the SSU r-protein.

The template DNA encoding an SSU r-protein should be operably linked by a promoter to the cDNA encoding an SSU r-protein. Regarding the structure of the template DNA encoding an SSU r-protein, portions other than the cDNA encoding an SSU r-protein are the same as those in the case of the template DNA encoding an LSU r-protein as described in the Section "2. Method for producing ribosomal LSU" above.

In addition, the template nucleic acid encoding an SSU r-protein may encode one SSU r-protein per molecule, or may encode two or more SSU r-proteins per molecule.

The template mRNA encoding an SSU r-protein may include a coding region (CDS) encoding the SSU r-protein, and a 5' untranslated region (5' UTR) may be linked to the 5' terminal side thereof and a 3' untranslated region (3' UTR) may be linked to the 3' terminal side thereof. Regarding the structure of the template mRNA encoding an SSU r-protein, portions other than CDS encoding the SSU r-protein are the same as in the case of the template mRNA encoding an LSU r-protein as described in the Section "2. Method for producing ribosomal LSU" above.

The nucleotide sequence of cDNA and CDS encoding each SSU r-protein is known. Examples of each cDNA encoding an SSU r-protein derived from *E. coli* include 30S r-protein S1: Gene ID: 945536, 30S r-protein S2: Gene ID: 947874, 30S r-protein S3: Gene ID: 947814, 30S r-protein S4: Gene ID: 947793, 30S r-protein S5: Gene ID: 947795, 30S r-protein S6: Gene ID: 948723, 30S r-protein S7: Gene ID: 947846, 30S r-protein S8: Gene ID: 947802, 30S r-protein S9: Gene ID: 949000, 30S r-protein S10: Gene ID: 947816, 30S r-protein S11: Gene ID: 947792, 30S r-protein S12: Gene ID: 947845, 30S r-protein S13: Gene ID: 947791, 30S r-protein S14: Gene ID: 947801, 30S r-protein S15: Gene ID: 947686, 30S r-protein S16: Gene ID: 947103, 30S r-protein S17: Gene ID: 947808, 30S r-protein S18: Gene ID: 948721, 30S r-protein S19: Gene ID: 947811, 30S r-protein S20: Gene ID: 944759, or 30S r-protein S21: Gene ID: 947577. These proteins are registered in a database provided by NIH.

In Production Method 2 of the present disclosure, when a ribosomal SSU containing a r-protein with a mutation introduced is produced, a desired mutation may be introduced into a template nucleic acid encoding the SSU r-protein.

The template nucleic acid encoding an SSU r-protein may be prepared by a known genetic engineering technique.

· SSU r-protein (in protein form) optionally used

Meanwhile, in Production Method 2 of the present disclosure, the SSU r-protein(s) (in a protein form) optionally used as a source of the SSU r-proteins constituting the ribosomal SSU may be separated from a naturally occurring ribosome, or may be produced using a genetic engineering technique.

[Source of SSU r-RNA used for formation of ribosomal SSU]

In Production Method 2 of the present disclosure, the source of SSU rRNA constituting a ribosomal SSU is an SSU rRNA (in an RNA form) or a template DNA encoding an SSU rRNA. When the SSU rRNA (in an RNA form) is used as a source of SSU rRNA, the SSU rRNA is directly assembled into the ribosomal SSU in a cell-free protein synthesis system. In addition, when the template DNA encoding an SSU rRNA is used as a source of SSU rRNA, the SSU rRNA transcribed from the template DNA in a cell-free protein synthesis system is directly assembled into the ribosomal SSU.

For example, in the case of producing a ribosomal SSU derived from *E. coli,* as a source of SSU rRNA, 16S rRNA or a template DNA encoding 16S rRNA is used.

In Production Method 2 of the present disclosure, preferable examples of the source of SSU rRNA constituting a ribosomal SSU include a template DNA encoding an SSU rRNA. A mutation can be easily introduced into the template DNA. Thus, when a template DNA encoding an SSU rRNA is used, a ribosomal SSU containing the SSU rRNA with a mutation introduced can be easily produced.

The template DNA encoding an SSU rRNA should be operably linked by a promoter to the DNA encoding an SSU rRNA. Regarding the structure of the template DNA encoding an SSU rRNA, portions other than the cDNA encoding an SSU rRNA are the same as those in the case of the template DNA encoding an LSU rRNA as described in the Section "2. Method for producing ribosomal LSU" above.

Also, a ribosomal SSU derived from *E. coli* may be produced. In this case, it is also possible to use, as the template DNA encoding an SSU rRNA, a template DNA encoding *E. coli* 16S rRNA or rrnB (operon encoding 16S rRNA, 23S rRNA, and 5S rRNA), which is an *E. coli* rRNA operon, or the like.

The nucleotide sequence of DNA encoding each SSU rRNA is known. For example, DNA encoding *E. coli*-derived SSU rRNA (16S rRNA) is registered as Gene ID: 948466 in a database provided by NIH.

The template DNA encoding an SSU rRNA may be double-stranded DNA, and may be either linear or circular such as a plasmid. The template DNA encoding an SSU rRNA may be prepared by a known genetic engineering technique.

In Production Method 2 of the present disclosure, when a ribosomal SSU containing a mutated SSU rRNA is produced, a desired mutation may be introduced into an SSU rRNA (in an RNA form) or a template DNA encoding an SSU rRNA used as a source.

[Formation of ribosomal SSU in cell-free protein synthesis system]

In Production Method 2 of the present disclosure, a source of SSU r-proteins (template nucleic acids encoding SSU r-proteins and optionally SSU r-protein(s) (in a protein form)) and a source of SSU rRNA (SSU rRNA (in an RNA form) or a template DNA encoding an SSU rRNA) may be incubated in a reaction solution of a cell-free protein synthesis system, and the SSU r-proteins and SSU rRNA are then assembled to form a ribosomal SSU.

The scheme of ribosomal SSU formation in Production Method 2 of the present disclosure is as follows. When the template nucleic acid encoding an SSU r-protein used as a source of SSU r-proteins is incubated in a reaction solution of a cell-free protein synthesis system, the template nucleic acid is transcribed/translated or translated to generate the SSU r-protein. In addition, when a template DNA encoding an SSU rRNA is used as a source of SSU rRNA, the template DNA is incubated in a reaction solution of a cell-free protein synthesis system and is transcribed to generate the SSU rRNA. Then, in the reaction solution of the cell-free protein synthesis system, (i) SSU r-proteins generated from template nucleic acids encoding the SSU r-proteins; (ii) an SSU r-protein(s) added in a protein form as a source of the SSU r-proteins, if necessary (when part of the SSU r-proteins contained in the ribosomal SSU is composed of the SSU r-proteins generated from the template nucleic acids); and (iii) SSU rRNA (added in an RNA form) and/or SSU rRNA generated from a template DNA encoding an SSU rRNA assemble to form a ribosomal SSU.

In Production Method 2 of the present disclosure, the reaction conditions in the cell-free protein synthesis system, the components to be added, and the like are the same as in the case of "2. Method for producing ribosomal LSU" except that the source of SSU r-proteins and SSU rRNA is used instead of the source of LSU r-proteins and LSU rRNAs.

The concentration of the template nucleic acid encoding an SSU r-protein in the reaction solution of the cell-free protein synthesis system may be appropriately adjusted to a range in which the ribosomal SSU can be formed according to the type and concentration of the source of the SSU rRNA contained in the reaction solution, the concentration of the ribosome used for translation in the cell-free protein synthesis system, and the like. However, from the viewpoint of efficiently forming the ribosomal SSU, the concentration of each template nucleic acid encoding an SSU r-protein is from 1 fM to 1 $\mu$M, preferably from 5 pM to 1 nM, and more preferably from 25 pM to 100 pM.

The SSU r-protein(s) (in a protein form) as a source of the SSU r-proteins may be used (part of the SSU r-proteins contained in the ribosomal SSU may be composed of the SSU r-proteins generated from the template nucleic acids). In this case, the concentration of each SSU r-protein added to the reaction solution of the cell-free protein synthesis system may be appropriately adjusted to a range in which the ribosomal SSU can be formed. For example, the concentration of each SSU r-protein added in the form of a protein is from 1 pM to 1 M, preferably from 1 nM to 1 mM, and more preferably from 10 nM to 1 $\mu$M.

The SSU rRNA (in an RNA form) may be used as a source of SSU rRNA in a cell-free protein synthesis system. In this case, the concentration of SSU rRNA added to the reaction solution of the cell-free protein synthesis system may be appropriately adjusted to a range in which the ribosomal SSU can be formed. For example, the concentration of SSU rRNA added in the form of an RNA is from 1 pM to 1 M, preferably from 1 nM to 1 mM, and more preferably from 10 nM to 1 $\mu$M.

The template DNA encoding an SSU rRNA may be used as a source of SSU rRNA in a cell-free protein synthesis system. In this case, the concentration of the template DNA added to the reaction solution of the cell-free protein synthesis system may be appropriately adjusted to a range in which the ribosomal SSU can be formed. However, from the viewpoint of efficiently forming the ribosomal SSU, the concentration of the template DNA is from 1 fM to 1 $\mu$M, preferably from 1 pM to 10 nM, and more preferably from 300 pM to 1 nM.

The time and temperature for incubating the reaction solution of the cell-free protein synthesis system are the same as in the above "2. Method for producing ribosomal LSU".

In the reaction solution after incubation, the generated ribosomal SSU is present alone. Alternatively, in a case where the origin is the same as or a closely related species with the endogenous ribosome, at least part of the generated ribosomal SSU is present in association with the endogenous ribosomal LSU to form a ribosome. The generated ribosomal SSU (including a state in which a ribosome associated with the endogenous ribosomal LSU is formed) can be purified by a known method such as centrifugation, chromatography, beads, or electrophoresis. In addition, a tag may be added to a template nucleic acid encoding an SSU r-protein, and when a template DNA encoding an SSU rRNA is used as a source of the SSU rRNA, a tag may be added to the template DNA. This also enables affinity purification. Examples of the tag include an MS2 tag, a FLAG tag, a His tag, a Strep tag, or a GST tag.

[Production of polymer using ribosomal SSU produced by Production Method 2 of the present disclosure]

The ribosomal SSU produced by Production Method 2 of the present disclosure may be used for producing a polymer in a cell-free synthesis system by forming a ribosome with a ribosomal LSU derived from the same species or a related species. In the production of a polymer in a cell-free synthesis system, the polymer may be synthesized from a template DNA or mRNA encoding a target polymer in a cell-free synthesis system using a ribosome including the ribosomal SSU produced by Production Method 2 of the present disclosure. The cell-free synthesis system may have components capable of transcribing and translating the template DNA or translating the template mRNA depending on the type of the polymer to be produced and the like. An example of the cell-free synthesis system includes a system having the same composition as that of the cell-free protein synthesis system.

The ribosome including the ribosomal SSU produced by Production Method 2 of the present disclosure can be used, for example, for synthesis of a peptide or protein using naturally occurring amino acids as a monomer.

In addition, in Production Method 2 of the present disclosure, a mutation can be easily introduced into an SSU r-protein and/or an SSU rRNA, and a ribosomal SSU having various catalytic activities can be produced. Therefore, the ribosome including the ribosomal SSU produced by Production Method 2 of the present disclosure may also be used for synthesis of a peptide or protein containing a non-natural amino acid, for example, while using a non-natural amino acid as at least one of monomers, depending on its activity. In addition, the ribosome including the ribosomal SSU produced by Production Method 2 of the present disclosure may also be used for synthesis of a non-natural polymer linked by an ester bond or a thioester bond while using, for example, a non-natural monomer that can be linked by an ester bond or a thioester bond, depending on its activity.

Further, a ribosomal LSU derived from the same species or a similar species may be added as necessary to the reaction solution of the cell-free protein synthesis system in which the ribosomal SSU is generated by performing Production Method 2 of the present disclosure. Furthermore, the polymer may also be produced by adding and incubating a template DNA or mRNA encoding the polymer and, if necessary, a monomer as a raw material.

<u>4. Method for producing ribosome</u>

Still another embodiment of the present disclosure provides a method for producing a ribosome in a cell-free protein synthesis system (hereinafter, sometimes referred to as Production Method 3 of the present disclosure). Production Method 3 of the present disclosure is a method for producing a ribosome, the method comprising the step of: assembling LSU r-proteins and LSU rRNAs and assembling SSU r-proteins and an SSU rRNA in a cell-free protein synthesis system to form a ribosome, wherein in the cell-free protein synthesis system, at least one of the LSU r-proteins or the SSU r-proteins is generated from a template nucleic acid encoding each corresponding protein. Hereinafter, Production Method 3 of the present disclosure will be described in detail.

[Ribosomal SSU]

The ribosome to be produced in Production Method 3 of the present disclosure may be derived from any of prokaryotes, eukaryotes, and archaea. Examples of the ribosome to be produced include a ribosome derived from a prokaryote, preferably a ribosome derived from *E. coli.*

In the present disclosure, the term "ribosome derived from a specific biological species" includes not only a naturally occurring ribosome composed of r-proteins and rRNAs possessed by the specific biological species, but also a variant thereof. For example, the ribosome derived from *E. coli* includes a naturally occurring ribosome derived from *E. coli* and a variant thereof. A ribosome subjected to various modifications in Production Method 3 of the present disclosure may be produced to provide a ribosome having modified catalytic activity, drug resistance, and the like.

In the present disclosure, examples of the variant of ribosome include a ribosome having at least one r-protein having a mutation and/or rRNA having a mutation.

In addition, as described above, it has been found that the ribosomal LSU can be formed even when the number of LSU r-proteins constituting the ribosomal LSU is one or more fewer than that of the naturally occurring ribosomal LSU. In addition, it has been found that the ribosomal SSU can be formed even when the number of SSU r-proteins constituting the ribosomal SSU is one or more fewer than that of the naturally occurring ribosomal SSU. Accordingly, in the present disclosure, examples of the variant of the ribosome include: a ribosome in which the number of r-proteins constituting the ribosomal LSU or SSU is one or more fewer than that of the naturally occurring ribosome; or a ribosome having at least one r-protein having a mutation and/or rRNA having a mutation, and in which the number of r-proteins constituting the ribosomal LSU or SSU is one or more fewer than that of the naturally occurring ribosome.

The number of LSU r-proteins constituting the ribosomal LSU in a ribosome to be produced by Production Method 3 of the present disclosure is as described in the Section "2. Method for producing ribosomal LSU". In addition, for example, the number of SSU r-proteins constituting the ribosomal SSU in a ribosome to be produced by Production Method 3 of the present disclosure is as described in the Section "3. Method for producing ribosomal SSU".

In addition, in the ribosome to be produced in Production Method 3 of the present disclosure, the anti-Shine-Dalgarno (ASD) sequence of the SSU rRNA may be set so as to be able to recognize a Shine-Dalgarno sequence (SD) that is not recognized by an ASD sequence of an SSU rRNA of an endogenous ribosome used for translation in the cell-free protein synthesis system in Production Method 3 of the present disclosure. Thus, the ribosome (hereinafter, nascent ribosome) produced by Production method 3 of the present disclosure and the endogenous ribosome are designed to recognize a different SD from each other. In this case, when a target polymer is produced using a nascent ribosome, one having an SD sequence that can be recognized by the nascent ribosome is used as a template DNA or a template mRNA encoding the target polymer. This makes it possible to suppress translation by the endogenous ribosomes and more specifically detect translation by the nascent ribosomes even in a state in which the endogenous ribosomes are mixed.

In addition, in the ribosome to be produced in Production Method 3 of the present disclosure, at least one of the rRNAs may be mutated to impart drug resistance. In a case where drug resistance is imparted to an LSU rRNA or SSU rRNA in this manner, when a target protein is produced in the presence of the drug by using a ribosome (nascent ribosome) produced by Production Method 3 of the present disclosure, translation by the endogenous ribosome can be suppressed and translation by the nascent ribosome can proceed even in a state where the endogenous ribosome is mixed. Specific examples of a mutation that confers drug resistance to an rRNA are as described in the Sections "2. Method for producing ribosomal LSU" and "3. Method for producing ribosomal SSU" above.

Further, in the ribosome to be produced in Production Method 3 of the present disclosure, at least one of the r-proteins may be mutated to impart drug resistance. In a case where drug resistance is imparted to an r-protein in this manner, when a target protein is produced in the presence of the drug by using a ribosome (nascent ribosome) containing the ribosomal SSU as produced by Production Method 3 of the present disclosure, translation by the endogenous ribosome can be suppressed and translation by the nascent ribosome can proceed even in a state where the endogenous ribosome is mixed. Specific examples of a mutation that confers drug resistance to an r-protein are as described in the section of "3. Method for producing ribosomal SSU" above.

[Source of r-proteins used for formation of ribosome]

In Production Method 3 of the present disclosure, at least one of the LSU r-proteins or SSU r-proteins constituting a ribosome is supplied from a template nucleic acid encoding each corresponding r-protein. When the number of r-proteins supplied from the template nucleic acids is less than the total number of r-proteins constituting the ribosome, an r-protein(s) (in a protein form) is used in addition to the template nucleic acids as a source of the r-proteins.

That is, all of the LSU r-proteins and SSU r-proteins contained in the ribosome may be composed of the r-proteins generated from the template nucleic acids. In this case, only the template nucleic acids may be added to the cell-free protein synthesis system as a source of the r-proteins, and the r-protein(s) (in a protein form) may not be added.

In addition, part of the LSU r-proteins and SSU r-proteins contained in the ribosome may be composed of the r-proteins generated from the template nucleic acids. In this case, the rest r-proteins contained in the ribosome are composed of r-proteins supplied in a protein form. That is, in this case, the r-proteins (in a protein form) may be added, as a source of the r-proteins, to the cell-free protein synthesis system together with the template nucleic acids.

In Production Method 3 of the present disclosure, at least one of the LSU r-proteins or SSU r-proteins constituting the ribosome may be supplied from the template nucleic acid(s).

In an embodiment of Production Method 3 of the present disclosure, the number of LSU r-proteins supplied from the template nucleic acids (the number of template nucleic acids used) among the LSU r-proteins constituting the ribosome is, for example, 1 or more, 2 or more, 5 or more, 10 or more, 15 or more, 20 or more, 25 or more, or 30 or more. As a preferred embodiment of Production Method 3 of the present disclosure, all of the LSU r-proteins constituting the ribosome are supplied from the template nucleic acids. Further, for example, a ribosome derived from *E. coli* may be produced. In this case, among the LSU r-proteins constituting the ribosome, the number of LSU r-proteins supplied from the template nucleic acids (the number of template nucleic acids used) is 1 or more, 2 or more, 5 or more, 10 or more, 15 or more, 20 or more, 25 or more, 26 or more, 27 or more, 28 or more, 29 or more, 30 or more, 31 or more, or 32 or more. In a preferred embodiment of producing a ribosome derived from *E. coli*, all of the LSU r-proteins constituting the ribosome are supplied from the template nucleic acids.

In an embodiment of Production Method 3 of the present disclosure, the number of SSU r-proteins supplied from the template nucleic acids (the number of template nucleic acids used) among the SSU r-proteins constituting the ribosome is, for example, 1 or more, 2 or more, 3 or more, 5 or more, 10 or more, 15 or more, or 20 or more. As a preferred embodiment of Production Method 3 of the present disclosure, all of the SSU r-proteins constituting the ribosome are supplied from the template nucleic acids. Further, for example, a ribosomal SSU derived from *E. coli* may be produced. In this case, the number of SSU r-proteins supplied from the template nucleic acids (the number of template nucleic acids used) among the SSU r-proteins constituting the ribosome is 1 or more, 2 or more, 3 or more, 5 or more, 10 or more, 15 or more, or 20 or more. In a preferred embodiment of producing a ribosome derived from *E. coli*, all of the SSU r-proteins constituting the ribosome are supplied from the template nucleic acids.

Hereinbelow, a template nucleic acid(s) encoding an r-protein(s) used as a source of the r-proteins constituting a ribosome and an r-protein(s) (in a protein form) optionally used as the source are as described in the Sections "2. Method for producing ribosomal LSU" and "3. Method for producing ribosomal SSU".

[Source of r-RNAs used for formation of ribosome]

In Production Method 3 of the present disclosure, the source of rRNAs constituting a ribosome is an rRNA(s) (in an RNA form) and/or a template DNA(s) encoding an rRNA(s). An rRNA(s) (in an RNA form) may be used as a source of rRNAs. In this case, in the cell-free protein synthesis system, the rRNA(s) is directly used for the ribosome formation. In addition, when the template DNA encoding an rRNA is used as a source of rRNAs, the rRNA transcribed from the template DNA in a cell-free protein synthesis system is directly used for the ribosome formation.

For example, in the case of producing a ribosome derived from *E. coli*, as a source of rRNAs, 5S rRNA or a template DNA encoding 5S rRNA, 23S rRNA or a template DNA encoding 23S rRNA, and 16S rRNA or a template DNA encoding 16S rRNA are used.

In Production Method 3 of the present disclosure, preferable examples of the source of rRNAs constituting a ribosome include a template DNA encoding an rRNA. A mutation can be easily introduced into the template DNA. Thus, when a template DNA encoding an rRNA is used, a ribosome containing the rRNA with a mutation introduced can be easily produced.

The sequence, structure, and the like of rRNA (in an RNA form) used as a source of rRNAs and a template DNA encoding an rRNA are as described in the Sections "2. Method for producing ribosomal LSU" and "3. Method for producing ribosomal SSU" above.

[Formation of ribosome in cell-free protein synthesis system]

In Production Method 3 of the present disclosure, a source of SSU r-proteins (template nucleic acids encoding r-proteins and optionally r-protein(s) (in a protein form)) and a source of rRNAs (rRNA(s) (in an RNA form) and/or a template DNA(s) encoding an rRNA(s)) may be incubated in a reaction solution of a cell-free protein synthesis system. This results in the assembly of the LSU r-proteins and the LSU rRNAs and the assembly of the SSU r-proteins and the SSU rRNA, and further results in the association of the ribosomal LSU and the ribosomal SSU to form a ribosome.

The scheme of ribosome formation in Production Method 3 of the present disclosure is as follows. When the template nucleic acid encoding an r-protein used as a source of r-proteins is incubated in a reaction solution of a cell-free protein synthesis system, the template nucleic acid is transcribed/translated or translated to generate the r-protein. In addition, when a template DNA encoding an rRNA is used as a source of rRNAs, the template DNA is incubated in a reaction solution of a cell-free protein synthesis system and is transcribed to generate the rRNA. Then, in the reaction solution of the cell-free protein synthesis system, (i) r-proteins generated from template nucleic acids encoding the r-proteins; (ii) an r-protein(s) added in a protein form as a source of the r-proteins, if necessary (when part of the r-proteins contained in the ribosome is composed of the r-proteins generated from the template nucleic acids); and (iii) an rRNA(s) (added in an RNA form) or rRNA(s) generated from a template DNA(s) encoding the rRNA(s) may be used. This results in the assembly of the LSU r-proteins and the LSU rRNAs and the assembly of the SSU r-proteins and the SSU rRNA to form a ribosomal LSU and a ribosomal SSU. Further, the formed ribosomal LSU and ribosomal SSU associate to form a ribosome.

In Production Method 3 of the present disclosure, the reaction conditions in the cell-free protein synthesis system, the components to be added, and the like are the same as in the Sections "2. Method for producing ribosomal LSU" and "3. Method for producing ribosomal SSU" except that the source of r-proteins (LSU r-proteins and SSU r-proteins) and the source of rRNAs (LSU rRNAs and SSU rRNA) are used. In addition, in Production Method 3 of the present disclosure, the concentration of each source, incubation time, temperature, and the like are also as described in the Sections "2. Method for producing ribosomal LSU" and "3. Method for producing ribosomal SSU" above.

The reaction solution after incubation contains generated ribosomes. Further, the produced ribosomes may have the same origin as the endogenous ribosomes. In this case, at least part of the generated ribosomal SSU may be in association with the endogenous ribosomal LSU to form a ribosome. Alternatively, at least part of the generated ribosomal LSU may be in association with the endogenous ribosomal SSU to form a ribosome. The generated ribosome may be purified by a known method such as centrifugation, chromatography, beads, or electrophoresis. In addition, a tag may be added to a template nucleic acid encoding an r-protein, and when a template DNA encoding an rRNA is used as a source of the rRNAs, a tag may be added to the template DNA. This also enables affinity purification. Examples of the tag include an MS2 tag, a FLAG tag, a His tag, a Strep tag, or a GST tag.

[Production of polymer using ribosome produced by Production Method 3 of the present disclosure]

The ribosome produced by Production Method 3 of the present disclosure may be used for production of a polymer in a cell-free synthesis system. In the production of a polymer in a cell-free synthesis system, the polymer may be synthesized from a template DNA or mRNA encoding a target polymer in a cell-free synthesis system using a ribosome produced by Production Method 3 of the present disclosure. The cell-free synthesis system may have components capable of transcribing and translating the template DNA or translating the template mRNA depending on the type of the polymer to be produced and the like. An example of the cell-free synthesis system includes a system having the same composition as that of the cell-free protein synthesis system.

The ribosome produced by Production Method 3 of the present disclosure can be used, for example, for synthesis of a peptide or protein using naturally occurring amino acids as a monomer.

In addition, in Production Method 3 of the present disclosure, a mutation can be easily introduced into an r-protein and/or an rRNA, and a ribosome having various catalytic activities can thus be produced. Therefore, the ribosome produced by Production Method 3 of the present disclosure may also be used for synthesis of a peptide or protein containing a non-natural amino acid, for example, while using a non-natural amino acid as at least one of monomers, depending on its catalytic activity. In addition, the ribosome produced by Production Method 3 of the present disclosure may also be used for synthesis of a non-natural polymer linked by an ester bond or a thioester bond while using, for example, a non-natural monomer that can be linked by an ester bond or a thioester bond, depending on its catalytic activity.

Further, to a reaction solution of a cell-free protein synthesis system in which the production method 3 of the present disclosure is carried out to generate ribosomes, a template DNA or mRNA encoding a target polymer and, if necessary, a monomer as a raw material may be added and incubated to produce the polymer.

Examples

The present disclosure is not limited to the description of the Embodiments and Examples according to the present invention. Various modifications that can be easily conceived by those skilled in the art without departing from the scope of the Claims are also included in the present invention. The contents of, for instance, the literatures disclosed herein are incorporated by reference in their entirety.

1. Test Method

1-1. Bacterial Strains and Plasmids

The chromosomal lacZ gene of BL21 Star™ (DE3) (Thermo Fisher Scientific, Waltham, MA, USA) was disrupted by recombination using Red recombinase (Reference 1). A specific protocol is briefly described below.

pKD46 encoding phage λ-Red recombinase was introduced into *E. coli*. Transformants were grown in 50 mL SOC medium containing 100 µg/ml ampicillin (Viccillin® for injection, Meiji Seika Pharma, Tokyo, Japan) and 10 mM L-(+)-arabinose (Nacalai Tesque, Kyoto, Japan). A fragment of the kanamycin resistance gene (kmr) was amplified using a primer set (H1P1 forward primer: 5'-GAAATTGTGAGCGGATAACAATTTCACACAGGAAACAGCTGTGTAGGCTGG AGCTGCTTC-3 ' (SEQ ID NO: 1) and P4H2 reverse primer: 5'-TTACGCGAAATACGGGCAGACATGGCCTGCCCGG TTATTAATTCCGGGGGAT CCGTCGACC-3' (SEQ ID NO: 2)) while pKD13 was used as a template, and introduced into *E. coli* by electroporation. Then, the electroporated cells were grown on LB agar medium containing 50 µg/ml of kanamycin monosulfate to select kanamycin resistant transformants. The resulting strain is designated as "BL 21 Star™ (DE3) lacZ::kmr".

The flippase (FLP) helper plasmid pCP20 was transformed into BL21 Star™ (DE3) lacZ::kmr, thereby eliminating the kmr gene. Since pCP20 carries a temperature-sensitive replicon and shows thermal induction of FLP synthesis, transformants were non-selectively cultured at 37°C. This was found to lead to a loss of kanamycin resistance. The resulting strain is designated as "BL 21 Star™ (DE3) lacZ::frt".

The *E. coli* rRNA operon, rrnB, was inserted into pET-41a (+). Genes encoding 54 different r-proteins were each cloned from the *E. coli* DH5α genome. Since r-protein S1 functions as a translation factor rather than as a structural component and can improve the protein yield (References 2 to 5), r-protein S1 was also included as one of r-proteins in this test. The expression of genes encoding the 54 different r-proteins was controlled by the consensus sequence of the T7 promoter (T7PCONS) (Reference 6) and the tandem repeat sequence of the adenosine 20 mer (EpsA20) (Reference 7), which improve the efficiency of transcription and translation. A mutation in a gene encoding an rRNA or an r-protein was introduced by PCR using a mutation-containing primers. The cell-free transcription and translation (CF-TXTL) plasmid was extracted and purified from *E. coli,* and used.

Table 1 shows the list of bacterial strains and plasmids used in this test.

[Table 1]

| E. coli strains | Features |
|---|---|
| DH5α | F⁻ φ80d*lacZ* ΔM15 Δ (*lacZYA-argF*) U169 *deoR reoA1 hsdR17* (r$_k$⁻, m$_k$⁺) *phoA supE44λ⁻ thi*-1 *gyrA96 relA*1 |
| BL21 Star™ (DE3) | F⁻ ompT hsd S$_B$ (r$_B$⁻, m$_B$⁻) gal dcm rne131 (DE3) |
| BL21 Star™ (DE3) *lacZ::kmr* | F⁻ ompT hsd S$_B$ (r$_B$⁻, m$_B$⁻) gal dem rne131 (DE3) *lacZ::kmr* |
| BL21 Star™ (DE3) *lacZ::frt* | F⁻ ompT hsd S$_B$ (r$_B$⁻, m$_B$⁻) gal dem rne131 (DE3) *lacZ::fit* |
| | |
| Plasmids | Features |
| pKD46 | λ-Red recombinase expression plasmid |
| pKD13 | Template plasmid for gene disruption |
| pCP20 | Temperature-sensitive FLP recombinase expression plasmid |
| pT7_wtASD_rRNA | T7P_LacO_rRNA (WT-ASD)_T7T |
| pT7_wtASD_rRNA (SpcR) | T7P_LacO_rRNA (WT-ASD, SpcR)_T7T |
| pT7_oASD_b_rRNA (SpcR) | T7P_LacO_rRNA (b-oASD, SpcR)_T7T |
| pT7_oASD_or1_rRNA (SpcR) | T7P_LacO_rRNA (or1-oASD, SpcR)_T7T |
| pT7_oASD_or1_rRNA (SpcR, CldR) | T7P_LacO_rRNA (or1-oASD, SpcR, CldR)_T7T |
| pT7_oASD_or4_rRNA (SpcR) | T7P_LacO_rRNA (or4-oASD, SpcR)_T7T |
| pT7_wtSD_sfGFP | T7P_LacO_WT-SD_sfGFP_T7T |
| pT7_oSD_a_sfGFP | T7P_LacO_a-oSD_sfGFP_T7T |
| pT7_oSD_b_sfGFP | T7P_LacO_b-oSD_sfGFP_T7T |
| pT7_oSD_c_sfGFP | T7P_LacO_b-oSD_sfGFP_T7T |
| pT7_oSD_d_sfGFP | T7P_LacO_b-oSD_sfGFP_T7T |
| pT7_oSD_or1_sfGFP | T7P_LacO_b-oSD_sfGFP_T7T |
| pT7_oSD_or4_sfGFP | T7P_LacO_b-oSD_sfGFP_T7T |
| pT7_oSD_i_sfGFP | T7P_LacO_b-oSD_sfGFP_T7T |
| pT7_wtSD_LacZ | T7P_LacO_WT-SD_LacZ_T7T |
| pT7PCONS_EpsA20_wtSD_LacZ | T7PCONS_LacO_WT-SD_LacZ |
| pT7_oSD_b_LacZ | T7P_LacO_b-oSD_LacZ_T7T |
| pT7_oSD_or1_LacZ | T7P_LacO_or1-oSD_LacZ_T7T |
| pT7_oSD_or4_LacZ | T7P_LacO_or4-oSD_LacZ_T7T |
| pT7_oSD_i_LacZ | T7P_LacO_i-oSD_LacZ_T7T |

(continued)

| Plasmids | Features |
|---|---|
| Plasmids encoding each r-protein and mutant r-protein | T7PCONS_LacO_EpsA20_WT-SD_r-protein |

| |
|---|
| T7P: T7 promoter<br>LacO: Lac operator<br>WT-ASD: wild-type anti-SD sequence<br>oASD: orthogonal anti-SD sequence<br>b-oASD, orl-oASD, or4-oASD: orthogonal anti-SD sequence indicated by b, or1, and or4 , respectively, in Table 3<br>WT-SD: wild-type SD sequence<br>a-oSD, b-oSD, c-oSD, d-oSD, or1-oSD, or4-oSD, j-oSD: orthogonal SD sequence indicated by a, b, c, d, or1, or4, and j, respectively, in Table 3<br>SpcR: spectinomycin resistance gene<br>CldR: clindamycin resistance gene<br>sfGFP: Superfolder GFP gene<br>LacZ: LacZ gene<br>T7T: T7 terminator sequence<br>T7PCONS: T7 promoter consensus sequence<br>EpsA20: adenosine tandem repeat sequence |

1-2. To prepare sonicated S12 cell extract

The sonicated S12 cell extract was prepared by a method modified from the previously described method (Reference 8). A specific protocol is briefly described below.

*Escherichia coli* (*E. coli*) was cultured in 200 mL of 2×YPTG medium at 37°C, and then pelleted by centrifugation. The cell pellet was resuspended in Buffer A (20 mM Tris, 100 mM NH$_4$Cl, 10 mM MgCl$_2$, 0.5 mM EDTA·2K, and 2mM DTT with the pH adjusted to 7.2 with HCl) and the suspended cells were disrupted with a frequency input of 20 kHz and 50% amplitude by using a Q125 Sonicator (Qsonica, Newtown, CT, USA). The energy input for sonication was set at 500 J for 1 mL of the cell suspension. The cell extract was centrifuged at 12,000 g for 10 minutes at 4°C to recover the supernatant. The obtained cell extract was flash frozen with liquid nitrogen and stored at -80°C until use.

1-3. To prepare French Press cell extract

The French Press S30 cell extract was prepared by modifying the previously reported method (References 9 and 10). A specific protocol is briefly described below. *E. coli* was cultured in 200 mL of 2×YPTG medium at 37°C, and then pelleted by centrifugation. The cell pellet was resuspended in Buffer A, and a Halt Protease Inhibitor Cocktail (Thermo Fisher Scientific) and an RNase Inhibitor (QIAGEN) were added thereto. Next, the cells were single-passed at a pressure of 20,000 psi by using an EmulsiFlex-C5 homogenizer (Avestin, Ottawa, Canada) to crush the cells. Further, an RNase inhibitor and dithiothreitol (DDT) were added, and centrifugation at 30,000 g for 30 minutes at 4°C was repeated twice. The collected supernatant was dialyzed 4 times in iSAT buffer (50 mM HEPES, 10 mM Mg Glutamate, 200 mM K Glutamate, 2mM DTT, 1 mM Spermidine, and 1 mM Putrescine with the pH adjusted to 7.6 with KOH), and then centrifuged and concentrated at 4,000 g using an Amicon Ultra-15 (3-kDa cutoff) (Merck Millipore, Burlington, MA, USA). The obtained cell extract was flash frozen with liquid nitrogen and stored at -80°C until use.

The French Press S150 cell extract was prepared by modifying the previously reported method (References 9 and 10). A specific protocol is briefly described below. BL21 Star™ (DE3) lacZ::frt carrying pT7_wtASD_rRNA was cultured in 1 L of 2×YPTG medium with 50 μg/mL kanamycin at 37°C until the OD600 was 0.5. Next, 0.1 mM isopropyl-β-D-thiogalactopyranoside (IPTG, Nacalai Tesque) was added and incubated, and the cells were then disrupted by a single pass at a pressure of 20,000 psi by using an EmulsiFlex-C5 homogenizer (Avestin) and centrifuged at 30,000 g for 30 min at 4°C. The collected supernatant was centrifuged at 90,000 g for 20 hours at 4°C. The collected supernatant was further centrifuged at 150,000 g for 3 hours at 4°C. The collected supernatant was then dialyzed 4 times in iSAT buffer, and then concentrated using an Amicon Ultra-15 (3-kDa cutoff) (Merck Millipore, Burlington, MA, USA). The obtained cell extract was flash frozen with liquid nitrogen and stored at -80°C until use.

1-4. To prepare cell extract containing ribosomes with artificial rRNA

The plasmid containing an artificial rRNA operon was introduced into BL21 Star™ (DE3) lacZ::frt. The obtained transformant was cultured in 2×YPTG medium at 37°C until the OD600 reached 0.7. The cultured cells were incubated with 0.1 mM or 1 mM IPTG (Nacalai Tesque) for 2 hours or 3 hours, and a cell extract was then prepared by the above protocol.

### 1-5. Transcription and translation in cell free system (CF-TXTL)

The test for checking the *in vitro* formation of an artificial ribosome was performed in a two-step reaction including a first reaction for forming an artificial ribosome and a second reaction for detecting the artificial ribosome formed.

The first reaction includes mixing and reacting at 37°C for 180 minutes, in 15 $\mu$L of the first reaction solution having the composition shown in Table 2, native ribosomes, the plasmids containing the r-protein genes, and the plasmid containing the rRNA operon. In the case of using a cell extract derived from a BL21 Star™ (DE3) strain or a derivative strain thereof induced by IPTG, T7 RNA polymerase (T7 RNAP, New England BioLabs, Ipswich, MA, USA) was not included in the first reaction solution. In order to quantify the ribosome concentration, kasugamycin (FUJIFILM Wako Pure Chemical Corporation) was added at 2 mM to the reaction solution 15 minutes after the start of the first reaction as in the previous report (Reference 11). It is known that kasugamycin, an antibiotic isolated from *Streptomyces kasugaensis,* inhibits translation initiation by interfering with the association of ribosomal subunits, but has no effect on the 70 S ribosome during translation or stopped (References 12 and 13).

[Table 2]

Components of first reaction solution

| Component contained | Manufacturer | Final concentration |
|---|---|---|
| *E. coli* cell extract | Homemade | |
| ATP | Sigma-Aldrich | 1.2 mM |
| GTP | Sigma-Aldrich | 0.85 mM |
| UTP | Sigma-Aldrich | 0.85 mM |
| CTP | Sigma-Aldrich | 0.85 mM |
| Folinic acid | Sigma-Aldrich | 34 ug/mL |
| *E. coli*-derived tRNA mixture | Roche | 170 ug/mL |
| Potassium glutamate hydrate | Sigma-Aldrich | 130 mM |
| ammonium glutamate | Roche | 10 mM |
| Magnesium glutamate tetrahydrate | Sigma-Aldrich | 14 mM |
| Amino acid mix (20AA) | Nacalai Tesque | 40 mM (2 mM for each amino acid) |
| Nicotinamide adenine dinucleotide (NAD) | Sigma-Aldrich | 0.33 mM |
| Coenzyme A sodium salt hydrate (CoA) | Sigma-Aldrich | 0.27 mM |
| Spermidine | Sigma-Aldrich | 1.5 mM |
| Putresein dihydrochloride | Sigma-Aldrich | 1 mM |
| Potassium oxalate monohydrate | Sigma-Aldrich | 4 mM |
| Phospho(enol)pyruvate-potassium salt (PEP) | Roche | 33 mM |
| T7 RNA polymerase (added in the case except for a cell extract derived from BL21 Star™ (DE3) strain or a variant strain thereof) | New England BioLabs | 0.8 U/$\mu$L |

For the second reaction, a second reaction solution was prepared which contained 3.6 $\mu$L of the cell extract, an antibiotic (200 $\mu$M spectinomycin, 20 $\mu$g/mL streptomycin, 3 mM clindamycin, or 240 nM thiostrepton), 3 nM of the reporter plasmid, and 1 mM of IPTG. Note that when the reporter plasmid expressed LacZ, the second reaction solution included 66 $\mu$M of 5-chloromethylfluorescein di-$\beta$-D-galactopyranoside (CMFDG; Invitrogen, Waltham, MA, USA) as a substrate for LacZ. In the second reaction, 15 $\mu$L of the second reaction solution was added to and mixed with 15 $\mu$L of the solution after the first reaction, and the signal of the reporter was then measured. In the second reaction, the antibiotic contained in the second reaction solution inactivates the native ribosomes, and expression of the sfGFP or LacZ reporter is induced by IPTG, so that a signal of the reporter due to the artificial ribosomes formed is generated. Since an SSU containing orl-oASD is formed while detecting an SSU, more specific detection is also possible by using a reporter containing or1-oSD. The signal of the reporter was quantified at $\lambda$ex = 485 nm and $\lambda$em = 535 nm by using a fluorescence microplate reader, Fluoroskan Ascent FL™ (Thermo Fisher Scientific) or Infinite® 200 PRO (TECAN, Mannedorf, Switzerland).

The first reaction and the second reaction were performed using a 96-well plate (polystyrene, solid bottom, half area, black-walled, Greiner Bio-One International GmbH, Kremsmunster, Austria).

The *in vitro* production of ribosomal SSU was specifically performed by the following procedure. First, native ribosomes, 21 different plasmids each containing one of 21 different SSU r-protein genes, and a plasmid containing an rRNA operon (rrnB) having orl-oASD and C1192U SpcR were mixed with a first reaction solution based on the S150 cell extract, and the mixture was incubated at 37°C for 180 minutes to perform a first reaction. Next, 15 μL of a second reaction solution containing a plasmid encoding LacZ or sfGFP, the S150 cell extract, and if necessary, CMFDG, spectinomycin, streptomycin, and the like was added to the solution after the first reaction and then mixed. The fluorescence of the reaction solution was measured by a bulk assay or a femtoliter droplet assay. In the bulk assay, the reporter signal was kinetically measured at λex = 485 nm and λem = 535 nm by using a Fluoroskan Ascent FL™ (Thermo Fisher Scientific) or Infinite® 200 PRO (TECAN) at 37°C for 120 minutes, 720 minutes, or 900 minutes. The femtoliter droplet assay technique will be described below.

The *in vitro* production of ribosomal LSU was specifically performed by the following procedure. First, native ribosomes, 33 different plasmids each containing one of 33 different LSU r-protein genes, and a plasmid containing an rRNA operon (rrnB) having orl-oASD, C1192U SpcR, and clindamycin resistance (CldR) due to the A2058U mutation of 23S rRNA (Reference 25) were mixed with a first reaction solution based on the S150 cell extract, and reacted at 37°C for 180 minutes. Next, 15 μL of a second reaction solution containing pT7_wtSD_LacZ or pT7PCONS_EpsA20_wtSD_LacZ, the S150 cell extract, and if necessary, CMFDG, clindamycin, thiostrepton, or the like was added to the solution after the first reaction and mixed. The fluorescence of the reaction solution was measured by a bulk assay in the same manner as in the case of the SSU.

### 1-6. To reconstitute SSU by iSAT

Reconstitution of an SSU by iSAT was performed with some modifications to the method described previously (References 9 and 10). Specifically, the procedure was performed as follows. In the first reaction, all proteins (TP70) of 70S ribosome and a plasmid containing an rRNA operon (rrnB) with orl-oASD and C1192U SpcR were mixed with a first reaction solution based on the S150 cell extract, and the mixture was incubated at 37°C for 180 minutes. Next, 15 μL of a second reaction solution containing pT7_oSD_or1_LacZ, CMFDG, spectinomycin, and the S150 cell extract was added to the solution after the first reaction and mixed. The fluorescence of the reaction solution was measured by a bulk assay. In the bulk assay, the reporter signal was kinetically measured using an Infinite® 200 PRO (TECAN) at 37°C for 720 minutes while λex = 485 nm and λem = 535 nm.

### 1-7. Femtoliter droplet assay

A mixed oil consisting of light mineral oil (Sigma-Aldrich Corporation, St. Louis, MO, USA), 4.5% sorbitan monooleate (Nacalai Tesque), and 0.5% Triton® X-100 (Nacalai Tesque) was provided. The solution after the second reaction and the mixed oil were mixed and a microtube (Maruemu Corporation, Osaka, Japan) was tapped 20 times to obtain an emulsion. The resulting emulsion was incubated at 37°C for 2 hours to 17 hours, and bright field and fluorescence images of the droplets were acquired over time using a confocal fluorescence microscope LSM700 (Carl Zeiss AG, Oberkochen, Germany). The 488 nm laser was focused using an oil-immersion objective lens (Plan-Apochromat 40x/1.4 Oil DIC M27, Carl Zeiss AG) and oil-immersion oil (ImmersolTM518F, Carl Zeiss AG).

### 1-8. Automated femtoliter droplet assay with deep learning

Since it is difficult to accurately grasp the characteristics of droplets from a bright field image, a deep learning model was devised for accurate automated analysis. Positive control fluorescent droplets were prepared using purified LacZ (FUJIFILM Wako Pure Chemical Corporation) and CMFDG, and 15 bright field images and fluorescent images including a total of 27580 fluorescent droplets were imaged. An illustrative (reference 14) pixel classification model was trained such that positive control fluorescence images as ground truth were each processed into a binarized image (droplets or background). Then, the droplets were separated from the background. A binarization model was constructed using a convolutional neural network architecture called U-Net (Reference 15). The FastAI library (Reference 16) was used in a virtual environment (Python 3.7, torch==1.4.0+cpu, torchvision==0.5.0+cpu) built on Anaconda. The model was trained using 13 ground-truth binarized images and corresponding bright-field images, and the remaining 2 images were used as test data. An encoder network, Resnet34, and load attenuation of 1e-2 were specified. A suitable learning rate was searched by the learn.lr_find() method, and the learning rate of 1e-4 was selected. The model was trained 20 epochs with slice (1e-4) and pct_start = 0.3 while using the fit_one_cycle() method. All the frozen layers were set free and the learning rate was searched again. The whole model was trained 100 epochs with slice (1e-4) and pct_start = 0.3 while using the fit_one_cycle() method. As a result, in the test data, the accuracy (the number of correctly classified pixels/the total number of pixels) reached 90% or higher. Using the trained U-Net deep learning model, a bright field image of droplets was processed into a binarized image in which white and black regions indicated the droplets and the background, respectively. The binarized images were subjected to particle analysis using ImageJ, and the results of the particle analysis were redirected to fluorescence images of the same origin. An automated analysis pipeline to support deep learning was used to automatically acquire the area, mean fluorescence intensity, minimum fluorescence intensity, maximum fluorescence intensity, integrated density, and centroid of each droplet in a scalable and objective manner.

## 2. To establish technology for detecting translation activity of artificial ribosome with high precision

### 2-1. To develop two-sided orthogonal translation system *in vitro*

Since ribosome synthesis is a recursive process in which an existing ribosome self-replicates a nascent ribosome, it is useful to have a specific assay for detecting the translational activity of the nascent ribosome in order to examine ribosome biosynthesis. Translation efficiency is mainly determined by RNA-RNA base pairing between the SD sequence of mRNA and the anti-SD (ASD) sequence of 16S rRNA. Thus, the generation of new SD and ASD sequences may lead to the development of various orthogonal translation systems useful for the detection of nascent ribosomes. There are four types of orthogonal translation systems (Fig. 10a). Among them, in order to specifically and highly sensitively detect the translational activity of a nascent ribosome, a two-sided orthogonal translation system is required. In the two-sided orthogonality, the following three conditions need to be satisfied. (1) mRNA with orthogonal SD (oSD) is not a substrate for native ribosomes, (2) nascent artificial ribosomes with orthogonal ASD (oASD) do not translate mRNA with wild-type SD (WT-SD), and (3) nascent artificial ribosomes with oASD efficiently translate mRNA with isogenic oSD. To date, it has been shown that some oASD-oSD pairs have two-sided orthogonality in *E. coli* (Reference 17), but it has been unknown whether there are oASD-oSD pairs that exhibit two-sided orthogonality *in vitro* (Reference 18).

To screen for oASD-oSD pairs exhibiting strong two-sided orthogonality in *E. coli* cell extract, the seven orthogonal ribosome-mRNA pairs shown in Table 3 (denoted as a, b, c, d, or1, or4, or j) were selected as candidates.

[Table 3]

| Name | Sequence around mRNA SD | Sequence around rRNA ASD | Reference |
|------|-------------------------|--------------------------|-----------|
| WT | UUUCAUC<u>GGAGG</u>CCGCAA**AUG** | <u>GU</u>.........AUCA<u>CCUCCUUA</u><br>722          1531 | PMID:4598299 |
| a | UUUCA<u>CACCACC</u>CGCAA**AUG** | <u>CG</u>.........AUCA<u>CUGUGG</u>UA | PMID:16408021 |
| b | UUUCA<u>CAACUG</u>CCCGCAA**AUG** | <u>CA</u>.........AUCA<u>CCGCAG</u>UA | PMID:16408021 |
| c | UUUCA<u>CAUCCC</u>UCCGCAA**AUG** | <u>CA</u>.........AUCA<u>UGGGAU</u>UA | PMID:16408021 |
| d | UUUCAU<u>CCCUCC</u>GCAA**AUG** | <u>GU</u>.........AUCA<u>UGGGAU</u>UA | PMID:16408021 |
| or1 | AUAGAAUUCUUAA<u>GUCUCG</u>AAAAAA**AUG** | <u>GU</u>.........AUCA<u>CGAGAC</u>UA | PMID:18522973 |
| or4 | AUAGAAUUCUUAA<u>GUUCCG</u>AAAAAA**AUG** | <u>GU</u>.........AUCA<u>CGGAAC</u>UA | PMID:18522973 |
| J | UUUUUC<u>CAACCACA</u>GAUCU**AUG** | <u>GU</u>.........AUCA<u>CUGUGG</u>UA | PMID:31477696 |

In SD sequences, the underlined portion indicates an SD sequence and the bold letters indicate a start codon; in ASD sequences, the underlined portion indicates an ASD sequence.
The SD sequence is present in 5'-untranslated region of mRNA.
The ASD sequence is present in 16S rRNA (at positions 722 and 1531).

First, an experimental scheme was devised to select an oSD sequence that does not interact with native ribosomes in *E. coli* cell extract (Fig. 10b). The pT7_wtSD_sfGFP or pT7_oSD_sfGFP reporter was mixed with two kinds of *E. coli* cell extract (sonicated S12 cell extract or French Press S30 cell extract). The results of bulk assays revealed that the 6 oSD sequences (b, c, d, or1, or4, j) showed no reporter signal and no or weak functional interaction with native ribosomes (Fig 10c). The results were comparable between the two kinds of *E. coli* cell extracts. Thus, the sonicated S12 cell extract which is easy to prepare was used for the following screening step. Further, orthogonality of the top four oSD sequences (b, or1, or4, j) was verified using a more sensitive LacZ reporter than the GFP reporter. As a result, 3 oSD sequences (b, or1, or4) showed no reporter signal in the bulk assay (Fig. 10d).

Next, an experimental scheme was designed to screen for an oASD-oSD pair exhibiting two-sided orthogonality in *E. coli* cell extract (Fig. 10e). First, examined were conditions for preparing an *E. coli* cell extract containing both native ribosomes and artificial ribosomes with oASD and C1192U spectinomycin resistance (SpcR) (Reference 19). BL21 Star™ (DE3) lacZ::frt was transformed with a plasmid containing rRNA with or without C1192U spectinomycin resistance (SpcR). These bacterial strains were cultured in 0.1 mM or 1 mM IPTG for 2 hours or 3 hours, and mixed with the pT7_wtSD_sfGFP reporter and the sonicated S12 cell extract with or without addition of 10 $\mu$M spectinomycin. As a result, under the condition of treatment with 0.1 mM IPTG for 3 hours, the strongest sfGFP production was observed in the presence of spectinomycin, indicating efficient expression of artificial rRNA (Fig. 11). Hence, in the following experiment, the condition of treatment with 0.1 mM IPTG for 3 hours was adopted.

Next, the optimal concentration of spectinomycin was examined. BL21 Star™ (DE3) lacZ::frt was transformed with a plasmid containing rRNA with or without C1192U spectinomycin resistance (SpcR). These bacterial strains were cultured in the presence of 0.1 mM IPTG for 3 hours, and the sonicated S12 cell extract and pT7_wtSD_LacZ reporter were mixed at various spectinomycin concentrations. As a result, it was demonstrated that when the spectinomycin concentration was 5 mM, the activity of the endogenous ribosome was strongly suppressed, and the translational activity of the artificial ribosome was specifically detected (Fig. 12). Therefore, in the following experiments, the spectinomycin concentration was set to 5 mM unless otherwise specified.

Next, the transcription/translation activity of the prepared *E. coli* cell extract was checked. A cell extract containing native ribosomes and artificial ribosomes with oASD (b, or1, or4) and SpcR was mixed with the pT7_wtSD_LacZ reporter. As a result, a reporter signal was detected in any case (Fig. 13a). This result has revealed that the *E. coli* cell extract can be functionally prepared.

Next, oASD sequences that do not interact with the pT7_wtSD_LacZ reporter were selected. Spectinomycin and a pT7_wtSD_LacZ reporter were mixed into an *E. coli* cell extract containing artificial ribosomes with each oASD (b, or1, or4). As a result, no reporter signal was detected, confirming that there was no or weak functional interaction between artificial ribosomes including oASD (b, or1, or4) and mRNA including WT-SD (Fig. 10f). In addition, an *E. coli* cell extract containing an artificial ribosome having each oASD (b, or1, or4) was mixed with the corresponding pT7_oSD_LacZ reporter and spectinomycin. In the case of the combination between pT7_oSD_or1_LacZ reporter and an artificial ribosome with orl-oASD and SpcR, a strong reporter signal was found to occur (Fig. 10g). Further, a control experiment was conducted to verify the two-sided orthogonality of a pair of orl-oASD and oSD. An *E. coli* cell extract containing native ribosomes and artificial ribosomes with WT-ASD and SpcR were mixed with spectinomycin and pT7_wtSD_LacZ or pT7_oSD_or1_LacZ reporter. As a result, ribosomes with wtASD and SpcR did not interact with the pT7_oSD_or1_LacZ reporter (Fig. 13b). Specifically, it was confirmed that the two-sided orthogonality of the orl-oASD and oSD pair was reliably obtained from the interaction between the oASD and oSD pair rather than from SpcR. That is, these results have revealed that the pair of orl-oASD and or1-oSD exhibits strong two-sided orthogonality in the *E. coli* cell extract.

## 2-2. To develop automated femtoliter droplet assay with deep learning

In order to examine the optimal reaction conditions for performing ribosome synthesis *in vitro,* a highly sensitive assay for detecting the translational activity of nascent ribosomes is important. Then, a femtoliter droplet assay was employed as an assay for detecting the translational activity of nascent ribosomes (Fig. 14a). In this assay, since a small amount of reaction solution is confined in a femtoliter droplet, the concentration of the enzyme and the substrate becomes very high, and the enzyme activity can be detected with high sensitivity (Reference 20). In addition, an automated pipeline with deep learning was developed for scalable and objective analysis using a femtoliter droplet assay (Fig. 14b). The analysis pipeline used a trained U-Net (Reference 15) deep learning model to convert bright field images of droplets into binarized images (droplets or background) with an accuracy of 90% or higher (Fig. 15). The binarized image was subjected to automated particle analysis using ImageJ (Reference 21) to determine the size, fluorescence intensity, and the like of each droplet.

The sensitivity of the femtoliter droplet assay was then evaluated. First, a cell extract containing 4.9 $\mu$M of an artificial ribosome having orl-oASD and SpcR and a control cell extract containing only native ribosomes were prepared.

Next, a control experiment was performed using a control cell extract containing only native ribosomes and a pT7_oSD_or1_LacZ reporter. The control cell extract was mixed with 5 nM pT7_oSD_or1_LacZ reporter. In this condition, no fluorescence from native ribosomes was detected in the bulk assay (Fig. 10d). Fluorescence from native ribosomes was observed in the femtoliter droplet assay (Fig. 16a), confirming that the femtoliter droplet assay is highly sensitive. When a femtoliter droplet assay was performed by mixing 5 nM pT7_oSD_or1_LacZ reporter and 100 $\mu$M spectinomycin in the control cell extract, it has been found that fluorescence derived from native ribosomes can be removed.

Next, a cell extract containing 4.9 $\mu$M artificial ribosomes with orl-oASD and SpcR was mixed with 5 nM pT7_oSD_or1_LacZ reporter and 100 $\mu$M spectinomycin to perform a femtoliter droplet assay. It has been found that the translational activity of an artificial ribosome having orl-oASD and SpcR can be specifically detected (Fig. 16b).

The sensitivity of the femtoliter droplet assay was then estimated.

The sonicated S12 cell extract containing native ribosomes and artificial ribosomes with orl-oASD and C1192U spectinomycin resistance (SpcR), and a control sonicated S12 cell extract containing only native ribosomes were diluted $10^3$ or $10^5$ fold; and the signal of the reporter was measured with femtoliter droplets in the presence of the pT7_oSD_or1_LacZ reporter and 100 $\mu$M spectinomycin (Fig. 14c). Since the threshold for the reporter signal, RFU, was 22, it was found that the femtoliter droplet assay enabled the detection of 49 pM artificial ribosome ($10^5$ fold dilution). Fig. 17 shows the results of creating a scatter plot of the average RFU with respect to the diameter of each droplet by using the data set of Fig. 14c.

Under 49 pM conditions, it is assumed that 1 $\mu$m diameter droplets each contain an average of 1.54 x $10^{-2}$ ribosomes. From the Poisson distribution equation, the probability that a 1 $\mu$m droplet contains k ribosomes is expressed by the following formula. According to the following formula, the probability of a droplet containing 0, 1, or 2 or more ribosomes is 0.9847, 0.0152, or 0.0001, respectively.

[Mathematical Formula 1]

$$P(k, \lambda) = \frac{\lambda^k e^{-\lambda}}{k!}$$

$\kappa$: the number of ribosomes
$\lambda$ : $1.54 \times 10^{-2}$

In the 49 pM artificial ribosome (dilution factor: $10^5$) data, 15544 droplets having a diameter of 0.5 to 1.5 $\mu$m were observed, and the number of fluorescent droplets among them was 17. That is, the ratio of the observed fluorescent droplets was 0.0011. In view of the all above, it is estimated that most fluorescent droplets (89%) each contain only one ribosome. The above results have demonstrated that the translational activity of the artificial ribosome can be detected at a single ribosome level with ultra-high sensitivity by automated analysis using the femtoliter droplet assay with deep learning.

3. To produce ribosome *in vitro*

3-1. To produce ribosomal SSU

A ribosomal SSU was produced *in vitro* using a two-sided orthogonal translation system and a femtoliter droplet assay. The ribosomal SSU was formed and checked in two separate reactions (Fig. 1a). In the first reaction, a ribosomal SSU was synthesized by transcribing an rRNA operon (rrnB) with orl-oASD and SpcR, transcribing and translating 21 different SSU r-protein genes, and assembling a ribosomal SSU in the S150 cell extract. In the second reaction, the translational activity of the nascent SSU was detected using the pT7_oSD_or1_LacZ reporter and 100 $\mu$M spectinomycin.

First, the conditions for the first reaction were set to 20 nM of native ribosomes, 0.25 nM each of the 21 different plasmids each containing one of the different 21 SSU r-protein genes, and 1 nM of the plasmid containing an rRNA operon with orl-oASD and C1192U spectinomycin resistance (SpcR). Here, no signal indicative of a ribosomal SSU was observed in the femtoliter droplet assay (Fig. 1b). Therefore, in the first reaction, each optimal concentration was searched, using the simplex-lattice design, in the range of 0-100 nM of native ribosomes, 0-0.5 nM each of the 21 different plasmids each containing one of 21 different SSU r-protein genes, and 0-3 nM of the plasmid containing an rRNA operon containing orl-oASD and SpcR. Then, a slight reporter signal was able to be detected by the femtoliter droplet assay (Fig. 1c).

The results of searching for the optimal concentration by the simple lattice design method have suggested that it is important for the reporter signal to reduce the concentrations of the plasmids containing the SSU r-protein genes and the plasmid containing the rRNA operon, and the structural components of the ribosome are expressed more efficiently than other genes. This may be because ribosome synthesis takes most of the energy required for cell division and the ribosome doubling time limits the cell doubling time, so that the ribosome synthesis is subject to strong selection pressure and should be highly efficient (References 22 and 23). Therefore, the concentration of each of the native ribosome, the 21 different plasmids each containing one of 21 different SSU r-protein genes, and the plasmid containing the rRNA operon with orl-oASD and SpcR in the first reaction was further examined. Specifically, the examination was performed in the range of more than 0 nM and 240 nM or less for the native ribosome, more than 0 nM and 0.15 nM or less for each of the 21 different plasmids each containing one of the 21 different SSU r-protein genes, and more than 0 nM and 0.9 nM or less for the plasmid containing the rRNA operon with orl-oASD and SpcR. Then, a strong reporter signal was able to be detected (Fig 1d). In particular, under optimized conditions of 80 nM native ribosomes, 0.05 nM each of the plasmids containing 21 different SSU r-protein genes, and 0.3 nM of the plasmid containing the rRNA operon, near saturation levels of reporter signal were observed in the femtoliter droplet assay (Fig. 1e). In addition, under this optimization condition, the reporter signal was able to be detected even in the bulk assay, and a ribosomal SSU was successfully formed much more efficiently than conventional iSAT (Fig. 1f).

In addition, the reporter signal was found to be derived from a nascent artificial SSU composed of r-proteins and rRNA newly synthesized from the plasmids. Specifically, in place of the gene encoding the wild-type 30S r-protein S12, the first reaction was carried out under the above optimization conditions using a gene encoding a K43T mutant of 30S r-protein S12, which mutant confers streptomycin resistance to ribosomes (References 18 and 24). In the second reaction, only spectinomycin or spectinomycin and streptomycin were mixed as antibiotics, and a reporter signal was detected by a bulk assay. As a result, the reporter signal was not affected even in the presence of spectinomycin and streptomycin only when the gene encoding the 30S r-protein S12 K43T mutant was used (Fig. 1 g). This result indicates that the nascent ribosomal SSU is composed mainly of newly synthesized rRNA and r-proteins. The above has revealed that a ribosomal SSU can be efficiently synthesized *in vitro* by simultaneously performing transcription of the rRNA operon, transcription and translation of the r-protein genes, and assembly of SSU.

### 3-2. To produce ribosomal SSU using 20 different SSU r-protein genes

A SSU was produced *in vitro* by excluding one r-protein from the 21 different SSU r-proteins (30S r-proteins) constituting the SSU. In the first reaction, in a first reaction solution based on the S150 cell extract, 80 nM of native ribosomes, 0.05 nM each of 20 different plasmids each containing one of 20 different SSU r-protein genes, and 0.3 nM of the plasmid containing the rRNA operon (rrnB) having orl-oASD and SpcR were mixed at each corresponding concentration, and the mixture was reacted at 37°C for 180 minutes. Next, in the second reaction, the signal of ribosomal SSU was detected by a bulk assay using the pT7_oSD_or1_LacZ reporter and spectinomycin.
As a result, when 30S r-protein S12 (rpsL) or 30S r-protein S16 (rpsP) was missing in the 21 different SSU r-proteins, the ribosomal SSU signal was weak, and the efficiency of ribosomal SSU formation was slightly reduced. However, even when one SSU r-protein other than 30S r-protein S12 was missing, the efficiency of ribosomal SSU formation was hardly affected (Fig. 2). That is, it was verified that a ribosomal SSU was successfully formed *in vitro* without using all the 21 SSU r-protein genes.

### 3-3. To produce ribosomal LSU

A ribosomal LSU was produced *in vitro* using a two-sided orthogonal translation system and a bulk assay. The experimental scheme is similar to that for the SSU (Fig 3a). In the first reaction, in a first reaction solution based on the S150 cell extract, the rRNA operon (rrnB) having orl-oASD, SpcR, and clindamycin resistance (CldR) due to the A2058U mutation of 23S rRNA (Reference 25) was transcribed, 33 different LSU r-protein genes were transcribed and translated, and a ribosomal LSU was assembled to synthesize a ribosomal LSU. In the second reaction, the translational activity of the nascent ribosomal LSU was detected by a bulk assay using the pT7_wtSD_LacZ reporter in the presence of 1.5 mM clindamycin.
In the first reaction, the native ribosome concentration was fixed at 80 nM, and the optimal concentrations of each of 33 different plasmids each containing one of 33 different LSU r-protein genes were searched within the range of 0-0.05 nM, and the optimal concentration of the plasmid containing the rRNA operon with orl-oASD, SpcR, and CldR was searched within the range of 0.2-0.4 nM. Under the condition of 0.01 nM of each plasmid containing the LSU r-protein gene and 0.4 nM of the plasmid containing the rRNA operon, the signal of ribosomal LSU was most strongly detected (Fig. 3b).
In addition, in the first reaction, the native ribosomes were used at 80 nM, and each optimal concentration was searched within the range of 0-0.015 nM for each of the 33 different plasmids each containing one of the 33 different LSU r-protein genes, and within the range of 0.6-1.5 nM for the plasmid containing the rRNA operon. Under the conditions that the native ribosome was at 80 nM, the plasmids containing 33 different LSU r-protein genes were each at 0.01 nM, and the plasmid containing the rRNA operon with orl-oASD, SpcR, and CldR was at 0.9 nM, the reporter signal derived from the synthesized ribosomal LSU was also able to be strongly detected by the bulk assay (Figs. 3c and 3d). Incidentally, the reporter signal of the nascent ribosomal LSU was lower than the reporter signal of the nascent ribosomal SSU (Figs. 3d and 1f). This may be because the nascent ribosomal LSU is capable of translating both LacZ and 33 different LSU r-proteins, and the ribosome with the A2058U CldR mutation retains only 30% or less of its translational activity in the presence of clindamycin (Reference 26).

### 3-4. Improved LSU signal

The LacZ reporter was changed from pT7_wtSD_LacZ to pT7PCONS_EpsA20_wtSD_lacZ in which the T7CONS_EpsA20 sequence was added upstream of the LacZ gene sequence, and ribosomal LSU formation was checked. Specifically, in the first reaction, in a first reaction solution based on the S150 cell extract, 80 nM of native ribosome, 0.01 nM of each of 33 different plasmids each containing one of the 33 different LSU r-protein genes, and 0.9 nM of the plasmid containing the rRNA operon (rrnB) having orl-oASD, SpcR, and A2058U clindamycin resistance (CldR) were reacted at each corresponding concentration. In the second reaction, the translational activity of the nascent LSU was then detected by a bulk assay using the pT7PCONS_EpsA20_wtSD_lacZ reporter or pT7_wtSD_LacZ reporter in the presence of 1.5 mM clindamycin.
As a result, in the case of using the pT7PCONS_EpsA20_wtSD_lacZ reporter, the signal of the LSU increased by nearly 5 times as compared to the case of the pT7_wtSD_LacZ reporter, and the RFU increased from slightly above 200 to slightly below 1000 (Fig. 3e).

### 3-5. To produce ribosomal LSU using 32 different LSU r-protein genes

An LSU was formed *in vitro* by excluding one r-protein from the 33 different r-proteins (50S r-proteins) constituting the ribosomal LSU. In the first reaction, in a first reaction solution based on the S150 cell extract, 80 nM of native ribosomes, 0.01 nM each of 32 different plasmids each containing one of 32 different LSU r-protein genes, and 0.9 nM of the plasmid containing the rRNA operon (rrnB) having orl-oASD, SpcR, and CldR were mixed at each corresponding concentration, and the mixture was reacted at 37°C for 180 minutes. Next, in the second reaction, the signal of ribosomal LSU was detected by a bulk assay using the pT7PCONS_EpsA20_wtSD_lacZ reporter and clindamycin.

As a result, when 50S r-protein L21 was missing in the 33 different LSU r-proteins, the ribosomal LSU signal was weak, and the efficiency of ribosomal LSU formation was slightly reduced. However, even when one LSU r-protein other than 50S r-protein L21 was missing, the efficiency of ribosomal LSU formation was hardly affected (Fig. 4). That is, it was revealed that a ribosomal LSU was successfully formed *in vitro* without using all the 33 LSU r-protein genes.

### 3-6. To produce LSU with thiostrepton resistance

Thiostrepton is known to inactivate an LSU by binding to 50S r-protein L11. An LSU was formed *in vitro* by excluding 50S r-protein L11 from the 33 different r-proteins (50S r-proteins) constituting the ribosomal LSU. Thus, an LSU with thiostrepton resistance was produced. In the first reaction, in a first reaction solution based on the S150 cell extract, 80 nM of native ribosomes, 0.01 nM each of 32 different plasmids each containing one of 32 different LSU r-protein genes excluding 50S r-protein L11, and 0.9 nM of the plasmid containing the rRNA operon (rrnB) having or1-oASD, SpcR, and CldR were mixed at each corresponding concentration, and the mixture was reacted at 37°C for 180 minutes. In the second reaction, the translational activity of the ribosomal LSU was then detected by a bulk assay using the pT7PCONS_EpsA20_wtSD_lacZ reporter in the presence or absence of thiostrepton.

As a result, when the activity of the native ribosome was measured under equivalent conditions, the signal of LSU disappeared in the presence of thiostrepton (Fig. 5a). An LSU signal was detected even in the presence of thiostrepton when the LSU was formed using plasmids each containing one of 32 different LSU r-protein genes excluding r-protein L11 gene (Fig. 5b). That is, the results have revealed that thiostrepton resistance can be imparted to an LSU by forming the ribosomal LSU *in vitro* without using the r-protein L11 gene.

### 3-7. Efficient ribosomal LSU formation using polysucrose

How polysucrose affected the formation of ribosomal LSU was examined. Specifically, in the first reaction, in a first reaction solution based on the S150 cell extract, 80 nM of native ribosomes, 0.01 nM each of 33 different plasmids each containing one of 33 different LSU r-protein genes, and 0.9 nM of the plasmid containing the rRNA operon (rrnB) having orl-oASD, SpcR, and CldR were mixed with polysucrose (Ficoll® 400) at a concentration of 0, 2, or 4.5 w/v%, and the mixture was reacted at 37°C for 180 minutes. Next, in the second reaction, the signal of ribosomal LSU was detected by a bulk assay using the pT7PCONS_EpsA20_wtSD_lacZ reporter and clindamycin.

As a result, in the case of adding polysucrose during the first reaction for forming a ribosomal LSU, the signal of LSU was higher than the case without adding polysucrose (Fig. 6). That is, it has been revealed that the efficiency of ribosomal LSU formation is improved by adding polysucrose during the first reaction for forming a ribosomal LSU.

### 3-8. To produce 70 S ribosome (simultaneous formation of SSU and LSU)

A ribosomal SSU and ribosomal LSU were simultaneously produced *in vitro* by the following procedure. In the first reaction, in a first reaction solution based on the S150 cell extract, 80 nM of native ribosomes, 0.01 nM each of 21 different plasmids each containing one of 21 different SSU r-protein genes, 0.01 nM each of 33 different plasmids each containing one of 33 different LSU r-protein genes, and 0.9 nM of the plasmid containing the rRNA operon (rrnB) having orl-oASD, C1192U SpcR, and A2058U CldR were first mixed at each corresponding concentration, and the mixture was reacted at 37°C for 180 minutes. Next, the reaction solution after the first reaction was divided into two. For one reaction solution, the SSU signal was measured by a bulk assay using the pT7_oSD_or1_LacZ reporter and spectinomycin, and for the other reaction solution, the LSU signal was measured by a bulk assay using the pT7PCONS_EpsA20_wtSD_lacZ reporter and clindamycin.

As a result, both the SSU and LSU reporter signals were detected, and it was confirmed that the SSU and the LSU were able to be reconstituted simultaneously *in vitro* (Fig. 7b).

3-9. To produce 70S ribosome with streptomycin resistance

A gene encoding a K43T mutant of 30S r-protein S12, which mutant confers streptomycin resistance to ribosomes, was used to produce a 70S ribosome with streptomycin resistance. In the first reaction, in a first reaction solution based on the S150 cell extract, 80 nM of native ribosomes, 0.01 nM each of 21 different plasmids each containing one of 21 different SSU r-protein genes (provided that the gene encoding 30S r-protein S12 used was a gene encoding the wild-type S12 or a K43T mutant), 0.01 nM each of 33 different plasmids each containing one of 33 different LSU r-protein genes, and 0.9 nM of the plasmid containing the rRNA operon (rrnB) having orl-oASD, C1192U SpcR, and A2058U CldR were mixed at each corresponding concentration, and the mixture was reacted at 37°C for 180 minutes. In the second reaction, the translational activity of the ribosomal SSU was then detected by a bulk assay using the pT7_oSD_or1_LacZ reporter in the presence of spectinomycin and in the presence or absence of streptomycin.
As a result, when the wild-type 30S r-protein S12 gene was used, no signal was detected in the presence of streptomycin, but when the gene encoding a K43T mutant of 30S r-protein S12 was used, a signal was detected even in the presence of streptomycin (Fig. 8). That is, these results have demonstrated that by using, as the 30S r-protein S12 gene, a gene encoding a K43T mutant of 30S r-protein S12, 70S ribosome having streptomycin resistance can be produced.

4. To produce and purify ribosome *in vitro*

In this test, in order to directly verify whether the nascent artificial ribosome produced in a test tube exhibits translational activity, the nascent artificial ribosome was purified to check the translational activity.

4-1. To produce SSU and LSU with streptavidin-binding aptamer

First, in order to purify a nascent artificial ribosome, a plasmid encoding rRNA with an Sb-aptamer inserted was constructed. The constructed plasmids are three types shown in Table 4.

[Table 4]

| Plasmid A [with 16S rRNA having an Sb-aptamer inserted] | Plasmid having or1-oASD and C1192SpcR and containing an rRNA operon (rrnB) with 16S rRNA having inserted an aptamer (Sb-aptamer) that binds to streptavidin. |
|---|---|
| Plasmid B [with 23S rRNA having an Sb-aptamer inserted] | Plasmid having or1-oASD and clindamycin resistance (CldR) due to 23S rRNA A2058U mutation and containing an rRNA operon (rrnB) with 23S rRNA having an Sb-aptamer inserted. |
| Plasmid C [with both 16S rRNA and 23S rRNA having an Sb-aptamer inserted] | Plasmid having or1-oASD, C1192U SpcR, and CldR and containing an rRNA operon (rrnB) with 16 S rRNA having an Sb-aptamer inserted and with 23S rRNA having an Sb-aptamer inserted. |

The sequence of the Sb-aptamer was set with reference to Reference 27. In plasmids A and C, the Sb-aptamer was inserted into helix 33a of 16S rRNA so that the function of 16S rRNA was not impaired, and in plasmids B and C, the Sb-aptamer was inserted into helix 25 of 23S rRNA so that the function of 23S rRNA was not impaired (references 28 and 29). The nucleotide sequence of plasmid A is shown in SEQ ID NO: 3. The Sb-aptamer is encoded at positions 3514 to 3568 of SEQ ID NO: 3. The nucleotide sequence of plasmid B is shown in SEQ ID NO: 4. The Sb-aptamer is encoded at positions 5011 to 5081 of SEQ ID NO: 4. The nucleotide sequence of plasmid C is shown in SEQ ID NO: 5. The Sb-aptamer is encoded at each of positions 3514 to 3568 and positions 5066 to 5136 of SEQ ID NO: 5.
Next, an SSU having 16S rRNA with an Sb-aptamer inserted and an LSU having 23S rRNA with an Sb-aptamer inserted were produced (Fig. 9a), and whether these actually had translational activity was checked.
The SSU production and the translational activity were checked by the following procedure. In the first reaction, in a first reaction solution based on the S150 cell extract, 80 nM of native ribosomes, 0.3 nM of plasmid A, and 0.05 nM each of the 21 different plasmids each containing one of 21 different SSU r-protein genes were mixed at each corresponding concentration, and the mixture was reacted at 37°C for 180 minutes. Next, in the second reaction, the translational activity of nascent ribosomal SSU was detected by a bulk assay using the pT7_oSD_or1_LacZ reporter and spectinomycin.
In addition, the LSU production and the translational activity were checked by the following procedure. In the first reaction, in a first reaction solution based on the S150 cell extract, 80 nM of native ribosomes, 0.9 nM of plasmid B, and 0.01 nM each of the 33 different plasmids each containing one of 33 different LSU r-protein genes were mixed at each corresponding concentration, and the mixture was reacted at 37°C for 180 minutes. In the second reaction, the translational activity of the nascent ribosomal LSU was then detected by a bulk assay using the pT7_wtSD_LacZ reporter in the presence of 1.5 mM clindamycin.

The results verified the presence of the translational activity of any of the SSU having 16S rRNA with an Sb-aptamer inserted and the LSU having 23S rRNA with an Sb-aptamer inserted (Fig. 9b).

4-2. To produce and purify SSU and LSU with streptavidin-binding aptamer

Next, an SSU having 16S rRNA with an Sb-aptamer inserted was produced and purified, an LSU having 23S rRNA with an Sb-aptamer inserted was produced and purified, and both were simultaneously produced and purified.

The SSU having 16S rRNA with an Sb-aptamer inserted was produced and purified by the following procedure. In a first reaction solution based on the S150 cell extract, 80 nM of native ribosomes, 0.3 nM of plasmid A, and 0.05 nM each of the 21 different plasmids each containing one of 21 different SSU r-protein genes were mixed at each corresponding concentration, and the mixture was reacted at 37°C for 180 minutes. Then, 250 $\mu$L of the obtained reaction solution, 50 $\mu$L of streptavidin agarose (Thermo Fisher Scientific), and 1.5 mL of ribosome dissociation buffer (20 mM HEPES-KOH, 1 mM magnesium glutamate, 200 mM potassium glutamate, 4 mM DTT, pH = 7.5) were mixed, and the mixture was incubated at 4°C for 16 hours. Next, the streptavidin agarose was washed seven times with 1 mL of ribosome dissociation buffer. The SSU with an Sb-aptamer was then eluted and purified from the streptavidin agarose by using the ribosome dissociation buffer with 25 mM biotin (Nacalai Tesque).

The LSU having 23S rRNA with an Sb-aptamer inserted was produced and purified by the following procedure. In a first reaction solution based on the S150 cell extract, 80 nM of native ribosomes, 0.9 nM of plasmid B, and 0.01 nM each of the 33 different plasmids each containing one of 33 different LSU r-protein genes were mixed at each corresponding concentration, and the mixture was reacted at 37°C for 180 minutes. The obtained reaction solution was used to purify the LSU having an Sb-aptamer under the same conditions as described above.

An SSU having 16S rRNA with an Sb-aptamer inserted and an LSU having 23S rRNA with an Sb-aptamer inserted were produced and purified simultaneously by the following procedure. In a first reaction solution based on the S150 cell extract, 80 nM of native ribosomes, 0.9 nM of plasmid B, 0.01 nM each of 21 different plasmids each containing one of 21 different SSU r-protein genes, and 0.01 nM each of 33 different plasmids each containing one of 33 different LSU r-protein genes were mixed at each corresponding concentration, and the mixture was reacted at 37°C for 180 minutes. The obtained reaction solution was used to purify the SSU having an Sb-aptamer and the LSU having an Sb aptamer under the same conditions as described above.

Here, rRNA was extracted using an RNeasy Mini Kit from the SSU having 16S rRNA with an Sb-aptamer inserted and/or the LSU having 23S rRNA with an Sb-aptamer inserted before and after the purification, and the rRNA was analyzed by electrophoresis. As a result, only the peak of 16S rRNA was observed from the SSU having 16S rRNA with an Sb-aptamer inserted after purification. This demonstrated that the SSU having 16S rRNA with an Sb-aptamer inserted was purified with high specificity (Fig. 9c). In addition, the purification was found to have high specificity (Fig. 9c) when the LSU having 23S rRNA with an Sb-aptamer inserted was produced and when the SSU having 16S rRNA with an Sb-aptamer inserted and the LSU having 23S rRNA with an Sb-aptamer inserted were simultaneously produced..

4-3. Translation activity of ribosomes purified

Next, a femtoliter droplet assay was applied to the nascent artificial ribosomes obtained by simultaneously producing and purifying the SSU having an Sb-aptamer and the LSU having an Sb-aptamer under the above conditions, and the translational activity thereof was detected. The femtoliter droplet assay was performed under the conditions in which 5 nM pT7_oSD_or1_LacZ reporter, 100 $\mu$M spectinomycin, and 1.5 mM clindamycin were mixed. The results showed that a nascent artificial ribosome purified after simultaneously producing the SSU having an Sb-aptamer and the LSU having an Sb-aptamer exhibited the translational activity in the presence of spectinomycin and clindamycin (Fig. 9d). This has proved that the purified nascent artificial ribosome was indeed functional.

5. List of References

Reference 1: Datsenko, K. A. et al. One-step inactivation of chromosomal genes in Escherichia coli K-12 using PCR products. Proc. Natl. Acad. Sci. U. S. A. 97, 6640-6645 (2000).

Reference 2: Boni, I. V. et al. Ribosome-messenger recognition: mRNA target sites for ribosomal protein S1. Nucleic Acids Res. 19, 155-162 (1991).

Reference 3: Komarova, A. V. et al. Protein S1 counteracts the inhibitory effect of the extended Shine-Dalgarno sequence on translation. RNA 8, 1137-1147 (2002).

Reference 4: Chen, S. S. et al. Characterization of the ribosome biogenesis landscape in E. coli using quantitative mass spectrometry. J. Mol. Biol. 425, 767-779 (2013).

Reference 5: Sheahan, T. et al. Ribosomal Protein S1 Improves the Protein Yield of an In Vitro Reconstituted Cell-Free Translation System. ACS Synth. Biol. 11, 1004-1008 (2022).

Reference 6: Shilling, P. J. et al. Improved designs for pET expression plasmids increase protein production yield in Escherichia coli. Commun Biol 3, 214 (2020).

Reference 7: Takahashi, S. et al. Translation enhancer improves the ribosome liberation from translation initiation. J. Am. Chem. Soc. 135, 13096-13106 (2013).

Reference 8: Kwon, Y.-C. et al. High-throughput preparation methods of crude extract for robust cell-free protein synthesis. Sci. Rep. 5, 8663 (2015).

Reference 9: Jewett, M. C. et al. In vitro integration of ribosomal RNA synthesis, ribosome assembly, and translation. Mol. Syst. Biol. 9, 678 (2013).

Reference 10: Fritz, B. R. et al. The impact of transcriptional tuning on in vitro integrated rRNA transcription and ribosome construction. Nucleic Acids Res. 42, 6774-6785 (2014).

Reference 11: Kempf, N. et al. A Novel Method to Evaluate Ribosomal Performance in Cell-Free Protein Synthesis Systems. Sci. Rep. 7, 46753 (2017).

Reference 12: Umezawa, H. et al. A NEW ANTIBIOTIC, KASUGSMYCIN. J. Antibiot. 18, 101-103 (1965).

Reference 13: Schluenzen, F. et al. The antibiotic kasugamycin mimics mRNA nucleotides to destabilize tRNA binding and inhibit canonical translation initiation. Nat. Struct. Mol. Biol. 13, 871-878 (2006).

Reference 14: Berg, S. et al. ilastik: interactive machine learning for (bio)image analysis. Nat. Methods 16, 1226-1232 (2019).

Reference 15: Ronneberger, O. et al. U-Net: Convolutional Networks for Biomedical Image Segmentation. in Medical Image Computing and Computer-Assisted Intervention - MICCAI 2015 234-241 (Springer International Publishing, 2015).

Reference 16: Howard, J. et al. Fastai: A Layered API for Deep Learning. Information 11, 108 (2020).

Reference 17: Rackham, O. et al. A network of orthogonal ribosome x mRNA pairs. Nat. Chem. Biol. 1, 159-166 (2005).

Reference 18: Shimojo, M. et al. In vitro reconstitution of functional small ribosomal subunit assembly for comprehensive analysis of ribosomal elements in E. coli. Commun Biol 3, 142 (2020).

Reference 19: Sigmund, C. D. et al. Antibiotic resistance mutations in 16S and 23S ribosomal RNA genes of Escherichia coli. Nucleic Acids Res. 12, 4653-4663 (1984).

Reference 20: Rondelez, Y. et al. Microfabricated arrays of femtoliter chambers allow single molecule enzymology. Nat. Biotechnol. 23, 361-365 (2005).

Reference 21: Schneider, C. A. et al. NIH Image to ImageJ: 25 years of image analysis. Nat. Methods 9, 671-675 (2012).

Reference 22: Maitra, A. et al. Bacterial growth laws reflect the evolutionary importance of energy efficiency. Proc. Natl. Acad. Sci. U. S. A. 112, 406-411 (2015).

Reference 23: Reuveni, S. et al. Ribosomes are optimized for autocatalytic production. Nature 547, 293-297 (2017).

Reference 24: Chumpolkulwong, N. et al. Effects of Escherichia coli ribosomal protein S12 mutations on cell-free protein synthesis. Eur. J. Biochem. 271, 1127-1134 (2004).

Reference 25: Cochella, L. et al. Isolation of antibiotic resistance mutations in the rRNA by using an in vitro selection system. Proc. Natl. Acad. Sci. U. S. A. 101, 3786-3791 (2004).

Reference 26: Hammerling, M. J. et al. In vitro ribosome synthesis and evolution through ribosome display. Nat. Commun. 11, 1108 (2020).

Reference 27: Bachler, M., Schroeder, R. & von Ahsen, U. StreptoTag: a novel method for the isolation of RNA-binding proteins. RNA 5, 1509-1516 (1999).

Reference 28: Leonov, A. A., Sergiev, P. V., Bogdanov, A. A., Brimacombe, R. & Dontsova, O. A. Affinity purification of ribosomes with a lethal G2655C mutation in 23 S rRNA that affects the translocation. J. Biol. Chem. 278, 25664-25670 (2003).

Reference 29: Golovina, A. Y., Bogdanov, A. A., Dontsova, O. A. & Sergiev, P. V. Purification of 30S ribosomal subunit by streptavidin affinity chromatography. Biochimie 92, 914-917 (2010).

**Claims**

1.  A method for producing a ribosomal large subunit in a cell-free protein synthesis system, the method comprising the step of:

    assembling ribosomal large subunit proteins and ribosomal large subunit RNAs in a cell-free protein synthesis system to form a ribosomal large subunit, wherein

in the cell-free protein synthesis system, at least one of the ribosomal large subunit proteins is generated from a template nucleic acid encoding each corresponding ribosomal large subunit protein.

2. The production method according to claim 1, wherein at least one of the ribosomal large subunit RNAs is generated from a template DNA encoding each corresponding ribosomal large subunit RNA in the cell-free protein synthesis system.

3. The production method according to claim 1 or 2, wherein the ribosomal large subunit is derived from *E. coli.*

4. The production method according to claim 3, wherein the ribosomal large subunit proteins constituting the ribosomal large subunit are free of 50S r-protein L11 and consists of 32 or less 50S r-proteins.

5. The production method according to claim 1 or 2, wherein any of the ribosomal large subunit RNAs is mutated to impart drug resistance.

6. The production method according to claim 1 or 2, wherein all the ribosomal large subunit proteins to be assembled are generated in the cell-free protein synthesis system from template nucleic acids encoding the ribosomal large subunit proteins.

7. A method for producing a polymer in a cell-free synthesis system, the method comprising:

a first step of assembling ribosomal large subunit proteins and ribosomal large subunit RNAs in a cell-free protein synthesis system to produce a ribosomal large subunit; and
a second step of synthesizing a polymer from a template nucleic acid encoding a sequence of the polymer in the cell-free synthesis system containing a ribosome having the ribosomal large subunit obtained in the first step, wherein
in the cell-free protein synthesis system of the first step, at least one of the ribosomal large subunit proteins is generated from a template nucleic acid encoding each corresponding ribosomal large subunit protein in the cell-free protein synthesis system.

8. A method for producing a ribosomal small subunit in a cell-free protein synthesis system, the method comprising the step of:

assembling ribosomal small subunit proteins and a ribosomal small subunit RNA in a cell-free protein synthesis system to form a ribosomal small subunit, wherein
in the cell-free protein synthesis system, at least two of the ribosomal small subunit proteins are generated from template nucleic acids encoding the ribosomal small subunit proteins.

9. The method for producing a ribosomal small subunit according to claim 8, wherein the ribosomal small subunit RNA is generated from a template DNA encoding the ribosomal small subunit RNA in the cell-free protein synthesis system.

10. The production method according to claim 8 or 9, wherein the ribosomal small subunit is derived from *E. coli.*

11. The production method according to claim 8 or 9, wherein an anti-Shine-Dalgarno sequence of the ribosomal small subunit RNA is set so as to be able to recognize a Shine-Dalgarno sequence that is not recognized by an anti-Shine-Dalgarno sequence of a ribosomal small subunit RNA of a ribosome used for translation in the cell-free protein synthesis system.

12. The production method according to claim 8 or 9, wherein at least one of the ribosomal small subunit RNA or the ribosomal small subunit proteins is mutated to impart drug resistance.

13. The production method according to claim 8 or 9, wherein all the ribosomal small subunit proteins to be assembled are generated in the cell-free protein synthesis system from template nucleic acids encoding the ribosomal small subunit proteins.

14. A method for producing a polymer in a cell-free synthesis system, the method comprising:

a first step of assembling ribosomal small subunit proteins and a ribosomal small subunit RNA in a cell-free protein synthesis system to produce a ribosomal small subunit; and

a second step of synthesizing a polymer from a template nucleic acid encoding a sequence of the polymer in the cell-free synthesis system containing a ribosome having the ribosomal small subunit obtained in the first step, wherein

in the cell-free protein synthesis system of the first step, at least two of the ribosomal small subunit proteins are generated from template nucleic acids encoding the ribosomal small subunit proteins.

15. A method for producing a ribosome in a cell-free protein synthesis system, the method comprising the step of:

assembling ribosomal large subunit proteins and ribosomal large subunit RNAs and assembling ribosomal small subunit proteins and a ribosomal small subunit RNA in a cell-free protein synthesis system to form a ribosome, wherein

in the cell-free protein synthesis system, at least one of the ribosomal large subunit proteins or the ribosomal small subunit proteins is generated from a template nucleic acid encoding each corresponding protein.

16. The production method according to claim 15, wherein the ribosome is derived from *E. coli.*

17. The production method according to claim 15 or 16, wherein all the ribosomal large subunit proteins to be assembled are generated in the cell-free protein synthesis system from template nucleic acids encoding the ribosomal large subunit proteins.

18. The production method according to claim 15 or 16, wherein all the ribosomal small subunit proteins to be assembled are generated in the cell-free protein synthesis system from template nucleic acids encoding the ribosomal small subunit proteins.

19. The production method according to claim 15 or 16, wherein at least one of the ribosomal large subunit RNAs or the ribosomal small subunit RNA is generated from a template DNA encoding each corresponding RNA in the cell-free protein synthesis system.

20. A method for producing a polymer in a cell-free synthesis system, the method comprising:

a first step of assembling ribosomal large subunit proteins and ribosomal large subunit RNAs and assembling ribosomal small subunit proteins and a ribosomal small subunit RNA in a cell-free protein synthesis system to form a ribosome; and

a second step of synthesizing a polymer from a template nucleic acid encoding a sequence of the polymer in the cell-free synthesis system containing the ribosome obtained in the first step, wherein

in the cell-free protein synthesis system of the first step, at least one of the ribosomal large subunit proteins or the ribosomal small subunit proteins is generated from a template nucleic acid encoding each corresponding protein.

FIG. 1

FIG. 2

FIG. 3

FIG. 4

FIG. 5

(a)

(b)

FIG. 6

FIG. 7

FIG. 8

FIG. 9

Extended Data Fig. 9

(a)

(b)

(c)

(c)

(d)

(c)

FIG. 10

FIG. 11

FIG. 12

(a)                                                    (b)

rRNA with SpcR                              rRNA without SpcR

Spectinomycin concentration                 Spectinomycin concentration

0 μM    10 μM    50 μM              0 μM    10 μM    50 μM
100 μM    500 μM    1,000 μM        100 μM    500 μM    1,000 μM
2,000 μM    5,000 μM    NC          2,000 μM    5,000 μM    NC

(c)

Spectinomycin concentration

☐ rRNA with SpcR
▨ rRNA without SpcR

FIG. 13

FIG. 14

FIG. 15

FIG. 16

**(a)**

| ASD | WT | WT |
| --- | --- | --- |
| Reporter | or1-oSD–LacZ | or1-oSD–LacZ |
| Spectinomycin | w/ | w/o |

**(b)**

| ASD | or1-oASD (SpcR) | or1-oASD (SpcR) |
| --- | --- | --- |
| Reporter | or1-oSD–LacZ | or1-oSD–LacZ |
| Spectinomycin | w/ | w/o |

FIG. 17

Dilution ratio $10^5$

**EP 4 567 115 A1**

## INTERNATIONAL SEARCH REPORT

| | International application No. |
|---|---|
| | **PCT/JP2023/028022** |

**A. CLASSIFICATION OF SUBJECT MATTER**

*C12N 15/31*(2006.01)i; *C12P 21/00*(2006.01)i; *C12P 21/02*(2006.01)i; *C07K 14/245*(2006.01)i
FI: C12N15/31 ZNA; C07K14/245; C12P21/02 C; C12P21/00 C

According to International Patent Classification (IPC) or to both national classification and IPC

**B. FIELDS SEARCHED**

Minimum documentation searched (classification system followed by classification symbols)

C12N15/31; C12P21/00; C12P21/02; C07K14/245

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | US 2022/0213467 A1 (BAR-ZIV, Roy et al.) 07 July 2022 (2022-07-07) | 1-3, 6-10, 13-20 |
| | claims 15, 16, paragraphs [0003], [0064], [0356], fig. 1A | |
| Y | | 11 |
| A | | 4, 5, 12 |
| Y | US 2017/0073381 A1 (JEWETT, Michael Christopher et al.) 16 March 2017 (2017-03-16) | 11 |
| | claims 1, 4 | |
| A | | 4, 5, 12 |
| A | LEVY, Michael et al., Cell-Free Gene Expression from DNA Brushes, Methods in Molecular Biology, 05 January 2022, vol. 2433, 135-149 | 1-20 |
| | entire text, all drawings | |
| A | LEVY, Michael et al., Boundary-Free Ribosome Compartmentalization by Gene Expression on a Surface, ACS Synth. Biol., 2021, 10, 609-619 | 1-20 |
| | entire text, all drawings | |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **22 September 2023** | **03 October 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

52

## INTERNATIONAL SEARCH REPORT

International application No.

**PCT/JP2023/028022**

| Box No. I | Nucleotide and/or amino acid sequence(s) (Continuation of item 1.c of the first sheet) |

1.  With regard to any nucleotide and/or amino acid sequence disclosed in the international application, the international search was carried out on the basis of a sequence listing:

     a.  ☑ forming part of the international application as filed:

         ☑ in the form of an Annex C/ST.25 text file.

         ☐ on paper or in the form of an image file.

     b.  ☐ furnished together with the international application under PCT Rule 13*ter*.1(a) for the purposes of international search only in the form of an Annex C/ST.25 text file.

     c.  ☐ furnished subsequent to the international filing date for the purposes of international search only:

         ☐ in the form of an Annex C/ST.25 text file (Rule 13*ter*.1(a)).

         ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

     "In the form of an Annex C/ST.25 text file" above should be understood as "in ST.26 format".

Form PCT/ISA/210 (continuation of first sheet) (January 2015)

International application No.

**PCT/JP2023/028022**

| Patent document cited in search report | | | Publication date (day/month/year) | Patent family member(s) | | | Publication date (day/month/year) |
|---|---|---|---|---|---|---|---|
| US | 2022/0213467 | A1 | 07 July 2022 | WO | 2021/059269 | A1 | |
| | | | | EP | 4034879 | A1 | |
| US | 2017/0073381 | A1 | 16 March 2017 | WO | 2015/184283 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MICHAEL C JEWETT et al.** In vitro integration of ribosomal RNA synthesis, ribosome assembly, and translation. *Mol Syst Biol.*, 25 June 2013, vol. 9, 678
- **SHIMOJO, M. et al.** In vitro reconstitution of functional small ribosomal subunit assembly for comprehensive analysis of ribosomal elements in E. coli. *Commun Biol*, 2020, vol. 3, 142
- *CHEMICAL ABSTRACTS*, 26873-85-8
- **DATSENKO, K. A. et al.** One-step inactivation of chromosomal genes in Escherichia coli K-12 using PCR products.. *Proc. Natl. Acad. Sci. U. S. A.*, 2000, vol. 97, 6640-6645
- **BONI, I. V. et al.** Ribosome-messenger recognition: mRNA target sites for ribosomal protein S1. *Nucleic Acids Res.*, 1991, vol. 19, 155-162
- **KOMAROVA, A. V. et al.** Protein S1 counteracts the inhibitory effect of the extended Shine-Dalgarno sequence on translation.. *RNA*, 2002, vol. 8, 1137-1147
- **CHEN, S. S. et al.** Characterization of the ribosome biogenesis landscape in E. coli using quantitative mass spectrometry.. *J. Mol. Biol.*, 2013, vol. 425, 767-779
- **SHEAHAN, T. et al.** Ribosomal Protein S1 Improves the Protein Yield of an In Vitro Reconstituted Cell-Free Translation System.. *ACS Synth. Biol.*, 2022, vol. 11, 1004-1008
- **SHILLING, P. J. et al.** Improved designs for pET expression plasmids increase protein production yield in Escherichia coli.. *Commun Biol*, 2020, vol. 3, 214
- **TAKAHASHI, S. et al.** Translation enhancer improves the ribosome liberation from translation initiation.. *J. Am. Chem. Soc.*, 2013, vol. 135, 13096-13106
- **KWON, Y.-C. et al.** High-throughput preparation methods of crude extract for robust cell-free protein synthesis.. *Sci. Rep.*, 2015, vol. 5, 8663
- **JEWETT, M. C. et al.** In vitro integration of ribosomal RNA synthesis, ribosome assembly, and translation.. *Mol. Syst. Biol.*, 2013, vol. 9, 678
- **FRITZ, B. R. et al.** The impact of transcriptional tuning on in vitro integrated rRNA transcription and ribosome construction.. *Nucleic Acids Res.*, 2014, vol. 42, 6774-6785
- **KEMPF, N. et al.** A Novel Method to Evaluate Ribosomal Performance in Cell-Free Protein Synthesis Systems.. *Sci. Rep.*, 2017, vol. 7, 46753
- **UMEZAWA, H. et al.** A NEW ANTIBIOTIC, KA-SUGSMYCIN. *J. Antibiot.*, 1965, vol. 18, 101-103
- **SCHLUENZEN, F. et al.** The antibiotic kasugamycin mimics mRNA nucleotides to destabilize tRNA binding and inhibit canonical translation initiation.. *Nat. Struct. Mol. Biol.*, 2006, vol. 13, 871-878
- **BERG, S. et al.** ilastik: interactive machine learning for (bio)image analysis.. *Nat. Methods*, 2019, vol. 16, 1226-1232
- U-Net: Convolutional Networks for Biomedical Image Segmentation. **RONNEBERGER, O. et al.** Medical Image Computing and Computer-Assisted Intervention - MICCAI. Springer International Publishing, 2015, vol. 2015, 234-241
- **HOWARD, J. et al.** Fastai: A Layered API for Deep Learning.. *Information*, 2020, vol. 11, 108
- **RACKHAM, O. et al.** A network of orthogonal ribosome x mRNA pairs.. *Nat. Chem. Biol.*, 2005, vol. 1, 159-166
- **SIGMUND, C. D. et al.** Antibiotic resistance mutations in 16S and 23S ribosomal RNA genes of Escherichia coli.. *Nucleic Acids Res.*, 1984, vol. 12, 4653-4663
- **RONDELEZ, Y. et al.** Microfabricated arrays of femtoliter chambers allow single molecule enzymology.. *Nat. Biotechnol.*, 2005, vol. 23, 361-365
- **SCHNEIDER, C. A. et al.** NIH Image to ImageJ: 25 years of image analysis.. *Nat. Methods*, 2012, vol. 9, 671-675
- **MAITRA, A. et al.** Bacterial growth laws reflect the evolutionary importance of energy efficiency.. *Proc. Natl. Acad. Sci. U. S. A.*, 2015, vol. 112, 406-411
- **REUVENI, S. et al.** Ribosomes are optimized for autocatalytic production.. *Nature*, 2017, vol. 547, 293-297
- **CHUMPOLKULWONG, N. et al.** Effects of Escherichia coli ribosomal protein S12 mutations on cell-free protein synthesis.. *Eur. J. Biochem.*, 2004, vol. 271, 1127-1134
- **COCHELLA, L. et al.** Isolation of antibiotic resistance mutations in the rRNA by using an in vitro selection system.. *Proc. Natl. Acad. Sci. U. S. A.*, 2004, vol. 101, 3786-3791
- **HAMMERLING, M. J. et al.** In vitro ribosome synthesis and evolution through ribosome display.. *Nat. Commun.*, 2020, vol. 11, 1108

- **BACHLER, M.** ; **SCHROEDER, R.** ; **VON AHSEN, U.** StreptoTag: a novel method for the isolation of RNA-binding proteins.. *RNA*, 1999, vol. 5, 1509-1516
- **LEONOV, A. A.** ; **SERGIEV, P. V.** ; **BOGDANOV, A. A.** ; **BRIMACOMBE, R.** ; **DONTSOVA, O. A.** Affinity purification of ribosomes with a lethal G2655C mutation in 23 S rRNA that affects the translocation.. *J. Biol. Chem.*, 2003, vol. 278, 25664-25670

- **GOLOVINA, A. Y.** ; **BOGDANOV, A. A.** ; **DONTSOVA, O. A.** ; **SERGIEV, P. V.** Purification of 30S ribosomal subunit by streptavidin affinity chromatography.. *Biochimie*, 2010, vol. 92, 914-917